# EUROPEAN PATENT APPLICATION

(11) **EP 4 643 889 A1**
(43) Date of publication of application: **05.11.2025**
(21) Application number: 23910821.0
(22) Date of filing: 28.12.2023
(51) Int. Cl.: A61K 47/68, A61K 35/17, A61P 35/00, C12N 5/0783

(54) **ANTI-5T4 ANTIBODY-NATURAL KILLER CELL CONJUGATE AND USE THEREOF**

(30) Priority: 30.12.2022 CN 202211728539
(71) Applicant: IMBIORAY (HANGZHOU) BIOMEDICINE CO., LTD., Hangzhou, Zhejiang 310052 (CN)
(72) Inventor: MIAO, Zhenwei, Hangzhou, Zhejiang 310052 (CN); ZHENG, Chen, Hangzhou, Zhejiang 310052 (CN); BIAN, Jinduo, Hangzhou, Zhejiang 310052 (CN); WU, Liqin, Hangzhou, Zhejiang 310052 (CN); SANG, Aowen, Hangzhou, Zhejiang 310052 (CN); SONG, Yan, Hangzhou, Zhejiang 310052 (CN); WANG, Guojun, Hangzhou, Zhejiang 310052 (CN); LU, Huan, Hangzhou, Zhejiang 310052 (CN); WANG, Cong, Hangzhou, Zhejiang 310052 (CN); LIU, Shizhou, Hangzhou, Zhejiang 310052 (CN); CHEN, Mingyue, Hangzhou, Zhejiang 310052 (CN); MEI, Shuang, Hangzhou, Zhejiang 310052 (CN)
(74) Representative: Nederlandsch Octrooibureau
(86) International application number: PCT/CN2023/142689
(87) International publication number: WO 2024/140904

(57) **Abstract**

The present application provides an anti-5T4 antibody-natural killer cell conjugate, a pharmaceutical composition comprising the conjugate, medical and pharmaceutical use of the cortjugate, and a preparation method therefor.

## Description

### CROSS-REFERECE TO RELATED APPLICATION

The present application claims priority to Chinese Patent Application No. 202211728539.X, filed on December 30, 2022, which is incorporated herein by reference in its entirety.

### TECHNICAL FIELD

The present application relates generally to the field of biopharmaceuticals. In particularly, the present application relates to an anti-5T4 antibody-natural killer cell conjugate, a pharmaceutical composition comprising the conjugate, medical and pharmaceutical uses of the conjugate, a preparation method therefor, and the like.

### BACKGROUND

According to the nationwide statistics on cancer in China released by the National Cancer Center of China in January 2019, there were about 3.929 million cases of malignancy and about 2.338 million deaths caused by malignancy nationwide in 2015. The cancer burden has continued to rise compared to historical data. In terms of incidence, lung cancer, liver cancer, upper digestive system tumor, colorectal cancer and female breast cancer remain the major malignancies in China. According to the nationwide statistics on cancer released by the National Cancer Center of China in February 2022, there were about 4.064 million cases of malignancy and about 2.4135 million deaths caused by malignancy nationwide in 2016. Compared to the 2015 data, the numbers of new cases and deaths from malignancies showed an upward trend, with lung cancer being the cancer with the highest incidence in China for many years.

Early symptoms of tumors are not manifested, some tumors are detected at an early stage but recur later, and tumors have high grades in malignancy, and due to these and other reasons, a significant proportion of patients has advanced tumors. Currently, common methods for the treatment of solid tumors in clinic include surgery, radiotherapy, chemotherapy, conventional biological therapy, and traditional Chinese medicine treatment, among others. These treatments are often accompanied by certain toxic and side effects. After surgical removal of a cancerous lesion, there may be complications of varying degrees and a risk of recurrence. Radiotherapy and chemotherapy are the treatments with the most toxic and side effects and can cause a certain degree of damages to the patient's body. Although traditional Chinese medicine treatment has relatively low toxic and side effects, it often only plays an auxiliary role. Conventional biological therapy also has many unavoidable toxic and side effects. However, for patients with advanced solid tumors, the majority of them are no longer suitable for surgery, radiotherapy, or concurrent chemoradiotherapy, and have failed standard systemic treatments.

Therefore, there is an urgent need in the art to explore new therapeutic methods for tumors.

### SUMMARY OF THE INVENTION

In a first aspect, the present application provides an antibody-natural killer cell (NK cell) conjugate, wherein the antibody is an anti-5T4 antibody or antigen-binding fragment thereof, and the anti-5T4 antibody or antigen-binding fragment thereof is conjugated to the NK cell via a linker.

In a second aspect, the present application provides a cell population comprising the antibody-natural killer cell (NK cell) conjugate of the first aspect.

In a third aspect, the present application provides a pharmaceutical composition comprising the antibody-natural killer cell (NK cell) conjugate of the first aspect or the cell population of the second aspect, and a pharmaceutically acceptable carrier.

In a fourth aspect, the present application provides the use of the antibody-natural killer cell (NK cell) conjugate of the first aspect or the cell population of the second aspect in the manufacture of a medicament for the treatment of a tumor in an individual, particularly a tumor with a high expression of 5T4 (5T4⁺) in tumor cells.

In a fifth aspect, the present application provides a method of treating a tumor in an individual, particularly a tumor with a high expression of 5T4 (5T4⁺) in tumor cells, the method comprising administering to the individual an effective amount of the antibody-natural killer cell (NK cell) conjugate of the first aspect, or the cell population of the second aspect, or the pharmaceutical composition of the third aspect.

As non-limiting examples, the present application provides the following embodiments:
1. An antibody-natural killer cell (NK cell) conjugate, wherein the antibody is an anti-5T4 antibody or antigen-binding fragment thereof, and the anti-5T4 antibody or antigen-binding fragment thereof is conjugated to the NK cell via a linker.
2. The antibody-natural killer cell (NK cell) conjugate of embodiment 1, wherein the anti-5T4 antibody or antigen-binding fragment thereof comprises:
   HCDR1 as set forth in SEQ ID NO: 5 or having at least 80%, 85%, 90%, 95% or 99% identity to the sequence set forth in SEQ ID NO: 5,
   HCDR2 as set forth in SEQ ID NO: 6 or having at least 80%, 85%, 90%, 95% or 99% identity to the sequence set forth in SEQ ID NO: 6,
   HCDR3 as set forth in SEQ ID NO: 7 or having at least 80%, 85%, 90%, 95% or 99% identity to the sequence set forth in SEQ ID NO: 7,
   LCDR1 as set forth in SEQ ID NO: 8 or having at least 80%, 85%, 90%, 95% or 99% identity to the sequence set forth in SEQ ID NO: 8,
   LCDR2 as set forth in SEQ ID NO: 9 or having at least 80%, 85%, 90%, 95% or 99% identity to the sequence set forth in SEQ ID NO: 9, and
   LCDR3 as set forth in SEQ ID NO: 10 or having at least 80%, 85%, 90%, 95% or 99% identity to the sequence set forth in SEQ ID NO: 10; and
   wherein the amino acid sequences of the HCDRs and the LCDRs are defined according to Kabat.
3. The antibody-natural killer cell (NK cell) conjugate of embodiment 1 or 2, wherein the amino acid sequence of the heavy chain variable region of the anti-5T4 antibody or antigen-binding fragment thereof is set forth in SEQ ID NO: 1 or has at least 80%, 85%, 90%, 95% or 99% identity to the sequence set forth in SEQ ID NO: 1, and the amino acid sequence of the light chain variable region of the anti-5T4 antibody or antigen-binding fragment thereof is set forth in SEQ ID NO: 3 or has at least 80%, 85%, 90%, 95% or 99% identity to the sequence set forth in SEQ ID NO: 3.
4. The antibody-natural killer cell (NK cell) conjugate of any one of embodiments 1-3, wherein the anti-5T4 antibody comprises a heavy chain variable region set forth in SEQ ID NO: 1, a heavy chain constant region set forth in SEQ ID NO: 2, a light chain variable region set forth in SEQ ID NO: 3, and a light chain constant region set forth in SEQ ID NO: 4.
5. The antibody-natural killer cell (NK cell) conjugate of any one of embodiments 1-4, wherein:
   the anti-5T4 antibody is an intact antibody, a single chain fragment variable (scFv) or a bispecific antibody; and/or
   the antigen-binding fragment of the anti-5T4 antibody is a Fab, a Fab', a Fv or a F(ab')₂; and/or
   the anti-5T4 antibody is a humanized antibody or a fully human antibody; and/or
   the anti-5T4 antibody is a monoclonal antibody; and/or
   the anti-5T4 antibody is of IgG1, IgG2 or IgG4 isotype; and/or
   the anti-5T4 antibody comprises a light chain constant region of kappa subtype or lambda subtype.
6. The antibody-natural killer cell (NK cell) conjugate of any one of embodiments 1-5, wherein the NK cell is CD16⁺ and/or NKG2D⁺, preferably CD16⁺NKG2D⁺.
7. The antibody-natural killer cell (NK cell) conjugate of any one of embodiments 1-6, wherein the conjugate comprises at least 90% of CD16⁺NKG2D⁺NK cells, preferably, meanwhile the conjugate comprises at least 95% of CD56+NK cells.
8. The antibody-natural killer cell (NK cell) conjugate of any one of embodiments 1-7, wherein
   the NK cell is obtained from *in vitro* culture and expansion of NK cells derived from peripheral blood mononuclear cells (PBMCs); or
   the NK cell is obtained from *in vitro* culture and expansion of NK cells derived from umbilical cord blood; or
   the NK cell is obtained from *in vitro* culture and expansion of an NK cell line; or
   the NK cell is obtained from *in vitro* induction, culture, and expansion of induced pluripotent stem cells (iPSCs) or mesenchymal stem cells (ESCs).
9. The antibody-natural killer cell (NK cell) conjugate of any one of embodiments 1 to 8, wherein the anti-5T4 antibody or antigen-binding fragment thereof is conjugated to the NK cell via a click chemical reaction of linkers.
10. The antibody-natural killer cell (NK cell) conjugate of embodiment 9, wherein the anti-5T4 antibody or antigen-binding fragment thereof is conjugated to the NK cell via a first linker conjugated to the anti-5T4 antibody or antigen-binding fragment thereof, and a second linker conjugated to the NK cell, with the first and second linkers conjugated to each other to form the antibody-NK cell conjugate.
11. The antibody-natural killer cell (NK cell) conjugate of embodiment 10, wherein the first linker is an active ester capable of forming a conjugation to a lysine residue of the antibody by a reaction converting an ester bond into an amide bond, for example, the active ester is a pentafluorophenyl ester, such as pentafluorophenyl piperidinate.
12. The antibody-natural killer cell (NK cell) conjugate of embodiment 11, wherein the first linker further comprises a carbon-carbon triple bond structure capable of undergoing a cyclization reaction with an azide group to form a five-membered ring of triazole, for example, the carbon-carbon triple bond structure is an octyne group.
13. The antibody-natural killer cell (NK cell) conjugate of embodiment 12, wherein the first linker is a dibenzoazacyclooctyne-glutaryl-amino polyethylene glycol-pentafluorophenyl acetylpiperidinate having the following structure: 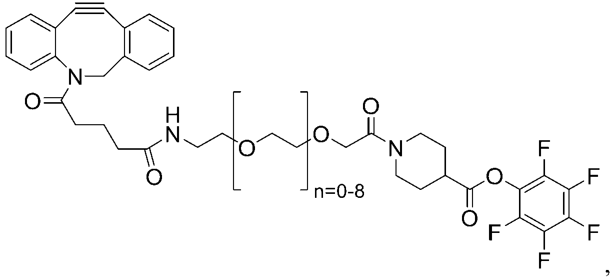 wherein n is an integer of 0-8.
14. The antibody-natural killer cell (NK cell) conjugate of embodiment 13, wherein the first linker is dibenzoazacyclooctyne-glutaryl-amino tetraethylene glycol-pentafluorophenyl acetylpiperidinate having the following structure: 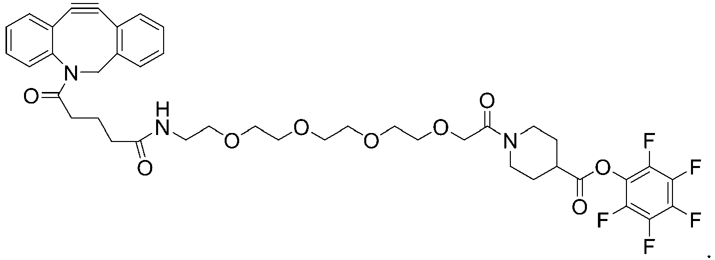
15. The antibody-natural killer cell (NK cell) conjugate of embodiment 10, wherein the second linker is azidoacetylcyclohexosamine, such as azidoacetylcyclogalactosamine or azidoacetylglucosamine.
16. The antibody-natural killer cell (NK cell) conjugate of embodiment 15, wherein the second linker is 1,3,4,6-oxo-tetraacetyl-2-azidoacetamide-2-deoxy-a,b-D-galactose having the following structure: 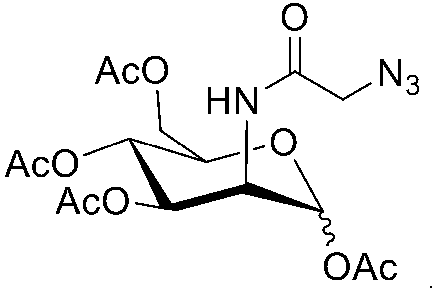
17. The antibody-natural killer cell (NK cell) conjugate of embodiment 16, wherein the second linker comprises at least 90% of the structure having a single α or β configuration.
18. A cell population comprising the antibody-natural killer cell (NK cell) conjugate of any one of embodiments 1-17.
19. The cell population of embodiment 18, wherein the count of CD3⁻CD56⁺CD16⁺ cells is at least 95%, preferably at least 98% with respect to the total cell count in the cell population, and/or the count of CD3⁻CD56⁺NKG2D⁺ cells is at least 95%, preferably at least 98% with respect to the total cell count in the cell population.
20. The cell population of embodiment 18 or 19, wherein:
   the count of CD3⁺CD56⁺ cells is no more than 5% with respect to the total cell count in the cell population; and/or
   the count of CD3⁻CD19⁺ cells is no more than 2% with respect to the total cell count in the cell population; and/or
   the counts of CD3⁺CD4⁺ and CD3⁺CD8⁺ cells are no more than 2% with respect to the total cell count in the cell population.
21. The cell population of any one of embodiments 18-20, wherein, by cell count, the antibody-natural killer cell (NK cell) conjugate is at least 90%, preferably at least 95%, more preferably at least 98%, most preferably at least 99% with respect to the total cell count in the cell population.
22. A pharmaceutical composition comprising the antibody-natural killer cell (NK cell) conjugate of any one of embodiments 1-17, or the cell population of any one of embodiments 18-21, and a pharmaceutically acceptable carrier.
23. The pharmaceutical composition of embodiment 22 comprising sodium chloride and/or human serum albumin.
24. The pharmaceutical composition of embodiment 22 or 23 comprising trehalose, sucrose, dextran, DMSO, or any combination thereof.
25. A pharmaceutical composition of any one of embodiments 22-24, for use in the treatment of a tumor in an individual, particularly a tumor with a high expression of 5T4 (5T4⁺) in tumor cells.
26. The pharmaceutical composition of embodiment 25, wherein the tumor is a solid tumor.
27. The pharmaceutical composition of embodiment 25, wherein the tumor is a malignant tumor.
28. The pharmaceutical composition of embodiment 25, wherein the tumor is a cancer.
29. The pharmaceutical composition of embodiment 28, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, ovarian cancer, renal cancer, bladder cancer, cervical cancer, colon cancer, liver cancer, prostate cancer, peritoneal cancer, hepatocellular cancer, glioblastoma, urinary tract cancer, rectal cancer, endometrial or uterine cancer, salivary gland cancer, squamous cell cancer (e.g., squamous cell carcinoma), vulvar cancer, thyroid cancer, anal cancer, penile cancer, melanoma, B-cell lymphoma, brain cancer, head and neck cancer, and metastases of the above cancers.
30. The pharmaceutical composition of embodiment 28, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, and ovarian cancer.
31. The pharmaceutical composition of embodiment 28, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, and colorectal cancer.
32. Use of the antibody-natural killer cell (NK cell) conjugate of any one of embodiments 1-17 or the cell population of any one of embodiments 18-21 in the manufacture of a medicament for the treatment of a tumor in an individual, particularly a tumor with a high expression of 5T4 (5T4⁺) in tumor cells.
33. The use of embodiment 32, wherein the tumor is a solid tumor.
34. The use of embodiment 32, wherein the tumor is a malignant tumor.
35. The use of embodiment 32, wherein the tumor is a cancer.
36. The use of embodiment 35, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, ovarian cancer, renal cancer, bladder cancer, cervical cancer, colon cancer, liver cancer, prostate cancer, peritoneal cancer, hepatocellular cancer, glioblastoma, urinary tract cancer, rectal cancer, endometrial or uterine cancer, salivary gland cancer, squamous cell cancer (e.g., squamous cell carcinoma), vulvar cancer, thyroid cancer, anal cancer, penile cancer, melanoma, B-cell lymphoma, brain cancer, head and neck cancer, and metastases of the above cancers.
37. The use of embodiment 35, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, and ovarian cancer.
38. The use of embodiment 35, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, and colorectal cancer.
39. A method for treating a tumor in an individual, particularly a tumor with a high expression of 5T4 (5T4⁺) in tumor cells, the method comprising administering to the individual an effective amount of the antibody-natural killer cell (NK cell) conjugate of any one of embodiments 1-17, or the cell population of any one of embodiments 18-21, or the pharmaceutical composition of any one of embodiments 22-31.
40. The method of embodiment 39, wherein the tumor is a solid tumor.
41. The method of embodiment 39, wherein the tumor is a malignant tumor.
42. The method of embodiment 39, wherein the tumor is a cancer.
43. The method of embodiment 42, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, ovarian cancer, renal cancer, bladder cancer, cervical cancer, colon cancer, liver cancer, prostate cancer, peritoneal cancer, hepatocellular cancer, glioblastoma, urinary tract cancer, rectal cancer, endometrial or uterine cancer, salivary gland cancer, squamous cell cancer (e.g., squamous cell carcinoma), vulvar cancer, thyroid cancer, anal cancer, penile cancer, melanoma, B-cell lymphoma, brain cancer, head and neck cancer, and metastases of the above cancers.
44. The method of embodiment 42, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, and ovarian cancer.
45. The method of embodiment 42, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, and colorectal cancer.

### BRIEF DESCRIPTION OF THE DRAWINGS

FIG. 1 shows the sequence and structural annotation of an exemplary anti-5T4 antibody of the present application.
FIG. 2 shows a schematic diagram of the structure of an exemplary anti-5T4 antibody-natural killer cell conjugate of the present application.
FIG. 3 shows results of a flow cytometric analysis of an IBR854 stock solution (Lot No. DSE20220711): CD56⁺, CD3⁻, CD16⁺, NKG2D⁺, CD19⁻.
FIG. 4 shows results of a flow cytometric analysis of an IBR854 formulation (Lot No. FE20220717): CD56⁺, CD3⁻, CD16⁺, NKG2D⁺, CD19⁻.
FIG. 5 shows results of a flow cytometric analysis of an UNK stock solution, the IBR854 stock solution, and the IBR854 formulation for the positivity rate and the geometric mean value of the conjugation.
FIG. 6 shows a schematic diagram of a preparation process for L2 linker.
FIG. 7 shows the structural formula of L2 linker, which is named as pentafluorophenyl (Z)-20-(1'-aza-2'-oxo-dibenzo[b,f]cyclo-7'-octynyl)-3,6,9,12-tetraoxa-15-aza-16,20-dioxodecanoyl piperidine-4-carboxylate.
FIG. 8 shows a schematic diagram of a preparation process for N1 linker.
FIG. 9 shows the structural formula of N1 linker, which is named as 1,3,4,6-tetra-O-acetyl-2-azidoacetylamido-2-deoxy-a,b-D-mannopyanose.
FIG. 10 shows a schematic diagram of the conjugation mechanism between L2 linker and the anti-5T4 antibody.
Figure 11 shows a schematic diagram of the conjugation mechanism between N1 linker and the NK cell.
FIG. 12 shows a schematic diagram of the structure of the UNK attached with N1 linker.
FIG. 13 shows a schematic diagram of the conjugation mechanism between the UNK and the L2-conjugated antibodies.
FIG. 14 shows a comparison of killing effects of IBR854, UNK cells, and NK cells on H1975 cells.
FIG. 15 shows a comparison of killing effects of IBR854, UNK cells, and NK cells on NCI-H292 cells.
FIG. 16 shows a comparison of killing effects of IBR854, UNK cells, and NK cells on NCI-H226 cells.
FIG. 17 shows a comparison of killing effects of IBR854, UNK cells, and NK cells on HCC827 cells.
FIG. 18 shows a comparison of killing effects of IBR854, UNK cells, and NK cells on Panc-1 cells.
FIG. 19 shows a comparison of killing effects of IBR854, UNK cells, and NK cells on BxPC3 cells.
FIG. 20 shows a comparison of killing effects of IBR854, UNK cells, and NK cells on MDA-MB-231 cells.
FIG. 21 shows a comparison of killing effects of IBR854, UNK cells, and NK cells on MDA-MB-468 cells.
FIG. 22 shows the levels of TraiL after IBR854 interacts with H292 tumor cells, with panel A and panel B showing the changes over time at an effector-to-target ratio of 10: 1, and panel C showing the changes over time at different effector-to-target ratios.
FIG. 23 shows the levels of FasL after IBR854 interacts with H292 tumor cells, with panel A and panel B showing the changes over time at an effector-to-target ratio of 10: 1, and panel C showing the changes over time at different effector-to-target ratios.
FIG. 24 shows tumor volume profiles for each group of animals in a test on mice with transplanted tumors of human lung cancer cell NCI-H292 M-NSG. * denotes P < 0.05, ** denotes P < 0.01, and *** denotes P < 0.001 as compared to the vehicle control group.
FIG. 25 shows tumor volume profiles for each group of animals in a test on mice with transplanted tumors of human breast cancer cell MDA-MB-468. * denotes P < 0.05, ** denotes P < 0.01, and *** denotes P < 0.001 as compared to the vehicle control group.
FIG. 26 shows the comparison of tumor weights of each group of animals on day 20 in a test on mice with transplanted tumors of human breast cancer cell MDA-MB-468. * denotes P < 0.05, ** denotes P < 0.01, and *** denotes P < 0.001 as compared to the vehicle control group.
FIG. 27 shows a comparison of killing effects of anti-5T4 antibody-natural killer cell conjugates with different L linkers, UNK cells, and NK cells on H292 cells, with the histograms for various effector-to-target ratios ordered from left to right as L3, L2, NK, and UNK.
FIG. 28 shows a graph showing killing effects of IBR854, NK cells, and a mixture of UNK cells+ the anti-5T4 conjugated antibody on LOVO cells over time.
FIG. 29 shows killing effects of IBR854, NK cells, and and the mixture of UNK cells+ the anti-5T4 conjugated antibody on LOVO cells for 55 hours.
FIG. 30 shows a graph showing killing effects of IBR854, NK cells, and the mixture of UNK cells + the anti-5T4 conjugated antibody on N87 cells over time.
FIG. 31 shows killing effects of IBR854, NK cells, and the mixture of UNK cells + the anti-5T4 conjugated antibody on N87 cells for 24 hours.

### DETAILED DESCRIPTION OF THE INVENTION

### Definition

Unless defined otherwise, all scientific and technical terms used herein have the same meaning as understood by a person of ordinary skill in the art. For definitions and terms in the art, a person skilled in the art can refer specifically to Current Protocols in Molecular Biology (Ausubel). Abbreviations for amino acid residues are the standard 3-letter and/or 1-letter codes used in the art to refer to one of the 20 common L-amino acids.

Although numerical ranges and parameter approximations are shown in broad ranges in the present application, the numerical values shown in the specific embodiments are described as accurately as possible. However, any numerical values inherently contain certain errors due to the standard deviation present in their respective measurements. Additionally, all ranges disclosed herein are to be understood as encompassing any and all subranges contained therein. For example, a range of "1 to 10" should be understood to encompass any and all subranges between the minimum value of 1 and the maximum value of 10, inclusive, i.e., all subranges starting with a minimum value of 1 or more, such as 1 to 6.1, and subranges ending with a maximum value of 10 or less, such as 5.5 to 10. Additionally, any reference referred to as "incorporated herein" should understood as being incorporated in its entirety.

As used herein, the term "individual" or "subject" refers to a mammal, such as a human, as well as other animals, such as wild animals, domestic animals, or experimental animals (e.g., gorillas, monkeys, rats, mice, rabbits, guinea pigs, woodchucks, or ground squirrels).

As used herein, the term "antigen" is a predetermined target to which an antibody can selectively bind. Examples of antigens include, but are not limited to, polypeptides, carbohydrates, nucleic acids, lipids, haptens, or other naturally occurring or synthetic compounds.

An "antibody", in its broad sense, refers to an immunoglobulin molecule that is capable of specifically binding to a target via at least one antigen recognition site located in the variable regions of the immunoglobulin molecule, and thus encompasses an intact antibody/full-length antibody, a single chain of an antibody, or any antigen binding fragment of an antibody (also referred to as an "antigen-binding portion"). When "antibody" and "antigen-binding fragment/antigen-binding portion" appear in the same context, an "antibody" can be understood as the entirety of the "antigen-binding fragment/antigen-binding portion", and they together correspond to the concept of an "antibody" in its broad sense.

The term "agonist antibody" is an antibody that initiates a reaction, for example, an antibody that mimics at least one functional activity of a target polypeptide. Agonist antibodies include antibodies that are ligand mimics, for example, wherein the ligand binds to a cell surface receptor, the binding induces cell signal transduction or activity via an intracellular cell signal transduction pathway, and wherein the antibody induces similar cell signal transduction or activation.

A "full-length/intact antibody" refers to a protein comprising at least two heavy (H) chains and two light (L) chains that are interconnected by disulfide bonds. Each heavy chain contains a heavy chain variable region (VH for short) and a heavy chain constant region. The heavy chain constant region contains three domains, CH1, CH2, and CH3. Each light chain contains a light chain variable region (VL for short) and a light chain constant region. The light chain constant region contains one domain, CL. The VH and VL regions can be further divided into a plurality of regions with high variability, referred to as complementarity determining regions (CDRs), interspersed with more conservative regions referred to as framework regions (FRs). Each VH or VL consists of three CDRs and four FRs, arranged from the amino terminus to the carboxy terminus in the following order: FR1, CDR1, FR2, CDR2, FR3, CDR3, and FR4. These variable regions of heavy and light chains contain binding domains that interact with antigens. The constant region of an antibody can mediate the binding of the immunoglobulin to tissues or factors in hosts, including various cells of the immune system (e.g., effector cells) and the first component of the classical complement system (Clq). A full-length/intact antibody can be any type of antibody, such as IgD, IgE, IgG, IgA or IgM (or subclasses of the above), but is not limited to any particular type. Immunoglobulins can be assigned to different classes depending on the amino acid sequences of the heavy chain constant domains of antibodies. Generally, immunoglobulins have five main classes, i.e., IgA, IgD, IgE, IgG and IgM. Several of these classes can be further classified into subclasses (isotypes), such as IgG1, IgG2, IgG3, IgG4, IgA1 and IgA2. Heavy chain constant domains corresponding to various immunoglobulin classes are referred to as α, δ, ε, γ and µ, respectively. Subunit structures and three-dimensional structures of the various immunoglobulin classes are well known. Chimeric or humanized antibodies are also encompassed by the antibodies according to the present application. It is well known to those skilled in the art that complementarity determining regions (CDRs, typically including CDR1, CDR2 and CDR3) are the subregions of the variable regions that have the most impact on the affinity and specificity of an antibody. CDR sequences in a VH or VL can be defined in multiple common ways, such as the Kabat definition, the IMGT definition, and the Chothia definition. For the variable region amino acid sequences of a given antibody, the CDR amino acid sequences in the VH and VL amino acid sequences can be determined according to different definitions. In embodiments of the present application, the CDR amino acid sequences are defined by the Kabat definition. For the variable region amino acid sequences of a given antibody, the CDR amino acid sequences in the variable region amino acid sequences can be analyzed in a variety of ways.

The term "humanized antibody" refers to an antibody obtained by grafting CDR sequences derived from another mammalian species, such as those in the mouse germline, onto human framework sequences. In order to retain binding affinity, some residues of the backbone segments (referred to as FR) can be modified. Humanized antibodies or fragments thereof according to the present application can be prepared by techniques known to those skilled in the art.

The term "chimeric antibody" refers to an antibody in which the variable region sequences are from one species and the constant region sequences are from another species, for example, an antibody in which the variable region sequences are from a murine antibody and the constant region sequences are from a human antibody. Chimeric antibodies or fragments thereof according to the present application can be prepared by using genetic recombination techniques. For example, a chimeric antibody can be produced by cloning a recombinant DNA comprising a promoter and sequences encoding variable regions of a non-human, particularly murine, monoclonal antibody according to the present application, and sequences encoding constant regions of a human antibody. Chimeric antibodies of the present application encoded by such recombinant genes will be, for example, a murine-human chimera, with its specificity determined by the variable regions derived from murine DNA and its isotype determined by the constant regions derived from human DNA.

The term "partially humanized antibody" refers to an antibody containing constant regions from a human and variable regions (including CDRs) from a non-human species (such as a mouse).

The term "semi-humanized antibody" is a type of humanized antibodies, and refers to an antibody in which one antibody chain comprises a murine variable region and the other antibody chain comprises a humanized variable region, i.e., a half-humanized antibody.

The term "monoclonal antibody" refers to an antibody obtained from a population of substantially homogeneous antibodies. That is, individual antibodies constituting the population are identical except that naturally occurring mutations may be present in a small number of individuals.

As used herein, the terms "antigen-binding fragment", "antigen-binding portion", and "antigen-binding region" are used interchangeably, and refer to parts of antibodies that contain amino acid residues interacting with antigens and conferring specificity and affinity to the binding agent, especially antibody fragments such as Fv, Fab, F(ab')₂, or Fab', or any fragment that can increase the half-life by chemical modification or by incorporation into liposomes, such as addition of poly(alkylene)glycols, such as polyethylene glycol ("pegylated") (referred to as pegylated fragments like Fv-PEG, scFv-PEG, Fab-PEG, F(ab')₂-PEG or Fab'-PEG) ("PEG" represents polyethylene glycol). Preferably, a functional fragment consists of or comprises a partial sequence of the heavy or light chain variable region of the antibody from which it is derived. The partial sequence is sufficient to retain the same binding specificity and sufficient affinity as the antibody from which it is derived. Such a functional fragment can comprise at least 5 amino acids, preferably 10, 15, 25, 50, and 100 contiguous amino acids of the antibody sequence from which it is derived. Examples of antigen-binding fragments include, but are not limited to, (1) Fab fragments, which can be monovalent fragments having VL-CL chains and VH-CH1 chains; (2) F(ab')₂ fragments, which can be divalent fragments having two Fab' fragments linked by disulfide bridges of the hinge region (i.e., dimers of Fab'); and (3) Fv fragments having VL and VH domains from a single arm of an antibody.

The term "single chain fragment variable (scFv)" refers to a single polypeptide chain formed by a VH domain and a VL domain that are linked via a peptide linker. A (scFv)₂ comprises two VH domains linked by a peptide linker and two VL domains that are combined with the two VH domains via disulfide bridges.

The term "Fc fragment", "Fc domain", "Fc moiety" or the like refers to a portion of the constant region of an antibody heavy chain, including the hinge region, the CH2 fragment and CH3 fragment of the constant region. The Fc region of the antibody may be subjected to engineering or modifications, including modifications related to effector functions, to such as reduce or eliminate antibody dependent cytotoxicity (ADCC) and/or complement-dependent cytotoxicity (CDC), which can be achieved by introducing one or more amino acid substitutions/mutations in the Fc region of the antibody.

The term "specific binding" refers to a non-random binding reaction between two molecules, such as the binding of an antibody to an antigenic epitope.

The term "bispecific antibody" refers to an antibody that has binding capacities for two antigenic epitopes. The two epitopes can be on different antigens or on the same antigen. Bispecific antibodies can adopt a variety of structural configurations. For example, a bispecific antibody can consist of two Fc fragments and two binding moieties fused to them, respectively (similar to a native antibody, except that the two arms bind to different antigenic targets or epitopes). An antigen binding moiety can be in the form of a single chain fragment variable (scfv) or a Fab fragment. The two different binding portions of the bispecific antibody each bind to the N-terminus of a single Fc fragment. The antigen-binding portions of the two arms may be configured in four combinations: scfv+Fab fragment, Fab fragment+scfv, scfv+scfv, and Fab fragment +Fab fragment. The Fc fragment may include mutations that ensure heavy chain heteropolymerization. The KIH (knob-in-hole) technique is a strategy for addressing heavy chain heteropolymerization. Generally, the KIH technique refers to modifying the amino acid sequence of the CH3 region to form a structure that facilitates pairing of heterologous incomplete antibodies, through which the normal antibody structure can be maintained as much as possible while constituting the bispecific antibody.

Generally, in order to prepare a monoclonal antibody or a functional fragment thereof, particularly a murine monoclonal antibody or a functional fragment thereof, one can refer to the techniques described in "Antibodies" manual (Harlow and Lane, Antibodies: A Laboratory Manual, Cold Spring Harbor Laboratory, Cold Spring Harbor NY, pp.726, 1988) or to the techniques for preparing a monoclonal antibody from hybridoma cells described by Kohler and Milstein (Nature, 256: 495-497, 1975).

The term "conservative variation" or "conservative amino acid substitutions" refers to substitutions that does not substantially affect or reduce the affinity of a protein. For example, an antibody can include up to about 1, up to about 2, up to about 5, up to about 10, or up to about 15 conservative substitutions, and specifically binds to the target antigen. The term "conservative variation" also includes the use of a substituted amino acid in place of the unsubstituted parent amino acid, as long as the antibody specifically binds to the target antigen.

The term "isolated" biological components (e.g., nucleic acids, proteins (including antibodies) or organelles) have been substantially isolated or purified from other biological components (i.e., other chromosomal and additional chromosomal DNA and RNA, proteins, and organelles) in the environment in which the components naturally occur (e.g., cells). Nucleic acids and proteins that have been "isolated" include those purified by standard purification methods. The term also includes nucleic acids and proteins that are prepared by recombinant expression in host cells, as well as chemically synthesized nucleic acids.

As used herein, the term "pharmaceutical composition" refers to a combination of at least one drug and, optionally, a pharmaceutically acceptable carrier or adjuvant that are combined together to achieve a particular purpose. In certain embodiments, the pharmaceutical composition includes a combination that is temporally and/or spatially separated, as long as it is capable of acting together to achieve the purpose of the present application. For example, the components contained in the pharmaceutical composition (e.g., antibody-cell conjugates according to the present application) can be administered to a subject together or separately. When the components contained in the pharmaceutical composition are separately administered to a subject, the components can be administered to the subject simultaneously or sequentially. The pharmaceutical composition according to the present application may include conventional components of a cell culture, particularly an NK cell culture, to maintain the activity of NK cells within the conjugate. The pharmaceutically acceptable carrier may include water, a buffered aqueous solution, an isotonic salt solution such as PBS (phosphate buffered saline), glucose, mannitol, dextrose, lactose, starch, magnesium stearate, cellulose, magnesium carbonate, 0.3% glycerol, hyaluronic acid, ethanol, or polyalkylene glycols such as polypropylene glycol, or triglycerides. The pharmaceutical composition or pharmaceutical preparation according to the present application may be administered via any suitable route, such as intravenous administration, intradermal, subcutaneous, intramuscular injection, or the like. The compositions according to the present application may comprise a wetting agent, an emulsifying agent, or a buffer substance as an additive.

As used herein, the term "a therapeutically effective amount" or "an effective amount" refers to a dose sufficient to show benefits to an individual being administered. The actual dose, rate, and timing of the administration will depend on the condition and severity of the disease in the individual to be treated. Prescription for the treatment (e.g., prescribed dose) is ultimately the responsibility of and dependent on the general practitioner or other physicians, and generally takes into account the disease being treated, the condition of individual patient, the delivery site, the administration method, and other factors known to the physicians.

The EC₅₀ value mainly refers to the concentration of a drug, antibody, toxin or the like that corresponds to 50% of the maximum biological effect after a specific period of exposure time. Pharmaceutically, in addition to being used to characterize the activation ability of agonists in *in vitro* experiments, it can also be used to denote the plasma concentration required to achieve half of the maximal biological effect *in vivo.* In some literature, EC₅₀ is also used to characterize the potency, including agonism and antagonism, of a compound at the cellular level. EC₅₀ values can be determined by methods such as ELISA.

The term "identity/homology/consistency" in the context of an amino acid or nucleic acid sequence is defined as the percentage of identical residues in an amino acid or nucleotide sequence variant that, after alignment and introduction of gaps, achieves maximum identity percentage, if desired. Methods and computer programs for sequence alignment are well known in the art.

As used herein, the term "tumor" refers to a neoplasm or solid lesion formed by abnormal cell growth. The tumor may be benign, precancerous, or malignant.

As used herein, the term "malignancy" refers to or describes a physiological condition of a mammal, typically characterized by unregulated cell growth. Exemplary malignancy include cancer, solid tumor, melanoma, sarcoma, hematological tumor, germinoma, and blastoma. More specific examples of malignancy include multiple myeloma, renal cancer, lung cancer including small cell lung cancer, non-small cell lung cancer, pulmonary adenocarcinoma, and pulmonary squamous cell carcinoma, bladder cancer, breast cancer, cervical cancer, colon cancer, hepatic carcinoma, stomach cancer including gastrointestinal cancer, prostate cancer, pancreatic cancer, peritoneal cancer, hepatocellular carcinoma, glioblastoma, ovarian cancer, liver cancer, urinary tract cancer, hepatoma, rectal cancer, colorectal cancer, endometrial or uterine cancer, salivary gland cancer, squamous cell cancer (e.g., squamous cell carcinoma), vulvar cancer, thyroid cancer, anal cancer, penile cancer, melanoma and B-cell lymphoma, brain cancer, head and neck cancer, and related metastases of the above cancers.

As used herein, the term "hematological tumor" refers to a tumor arising from uncontrolled growth and proliferation of abnormal cells, which typically originating in bone marrow, which is where blood cells are produced. Exemplary hematological tumors include various types of leukemia, multiple myeloma, and malignant lymphoma. More specific examples of hematological tumors include acute lymphoblastic leukemia (ALL), chronic lymphocytic leukemia (CLL), acute myelocytic leukemia (AML), chronic myelocytic leukemia (CML), hairy cell leukemia (HCL), T-cell lymphocytic leukemia, large granule lymphocytic leukemia, juvenile myelomonocytic leukemia, B-cell lymphocytic leukemia, Burkitt leukemia and adult T-cell leukemia, non-Hodgkin lymphoma, B-cell lymphoma, small lymphocytic lymphoma, lymphoplasmacytic lymphoma, primary macroglobulinemia (Waldenström macroglobulinemia), splenic marginal zone lymphoma, plasmacytoma, extranodal marginal zone B-cell lymphoma, MALT lymphoma, intranodal marginal zone B-cell lymphoma (NMZL), follicular lymphoma, mantle cell lymphoma, diffuse large B-cell lymphoma, mediastinal (thymus) large B-cell lymphoma, intravascular large B-cell lymphoma, primary effusion lymphoma, Burkitt lymphoma, B-cell chronic lymphocytic lymphoma, classical Hodgkin lymphoma, nodular lymphocyte-predominant Hodgkin lymphoma, adult T-cell lymphoma, extranodal nasal NK/T-cell lymphoma, enteropathic T-cell lymphoma, hepatosplenic T-cell lymphoma, NK-lymphoblastic lymphoma, mycosis fungoides, Sézary syndrome, primary cutaneous CD30 positive T-cell lymphoproliferative disease, primary cutaneous anaplastic large-cell lymphoma, lymphoma-like papulosis, angioimmunoblastic T-cell lymphoma, peripheral T-cell lymphoma not otherwise specified, and anaplastic large-cell lymphoma.

As used herein, the term "solid tumor" refers to a tangible mass that can be detected by clinical examination such as X-ray, CT scan, B-ultrasound, or palpation. Solid tumors that have been diagnosed and treated clinically are classified into two types, malignant and benign. Malignant solid tumors include: Hodgkin lymphoma in children: lymphocyte-predominant subtype, nodular sclerosis subtype, mixed cell subtype, lymphocyte-depleted subtype; non-Hodgkin lymphoma in children: prolymphoblastic lymphoma, small non-cleaved cell lymphoma (Burkitt/non-Burkitt lymphoma), diffuse large B-cell lymphoma, anaplastic large cell lymphoma, and the like; renal tumors in children: nephroblastoma (Wilms tumor), renal clear cell carcinoma, rhabdoid tumor of the kidney, renal clear cell sarcoma, renal primitive neuroectodermal tumor, and the like; neuroblastoma in children: neuroblastoma, ganglioneuroblastoma, ganglioneuroma; extracranial germinoma in children: mature teratoma, immature teratoma, endodermal sinus tumor (yolk sac tumor), seminoma, dysgerminoma, choriocarcinoma, embryonic carcinoma, and the like; osteosarcoma and chondrosarcoma; rhabdomyosarcoma in children: embryonal, alveolar, polymorphic, and the like; soft tissue sarcomas in children: fibrosarcoma, malignant fibrous histiocytoma, liposarcoma, leiomyosarcoma, angiosarcoma, lymphangiosarcoma, malignant schwannoma, acinar soft tissue sarcoma, epithelioid sarcoma, clear cell sarcoma, malignant melanoma, synovial sarcoma, desmoplastic small round cell tumor, and the like; Ewing family sarcoma: Ewing sarcoma, primary neuroectodermal tumor; liver tumors in children: hepatoblastoma (embryonal, fetal, undifferentiated), hepatocellular carcinoma; retinoblastoma; other tumors: medulloblastoma of the posterior cranial fossa, nasopharyngeal carcinoma, papillary thyroid carcinoma, thymoma, lung blastoma, pancreatic blastoma, islet cell tumor, ileocecal carcinoid, mesothelioma, and the like. Benign solid tumors include lymphangiomas, hemangiomas, thyroglossal cysts, and the like.

For the treatment of tumors, adoptive cellular immunotherapy has become a hot topic in research in China and worldwide, and has achieved good results in clinical tumor trials. Adoptive cellular immunotherapy is a therapy by isolating autologous or allogeneic immune cells, activating or genetically modifying them *in vitro,* expanding them to a sufficient quantity of immune cells with anti-tumor activity, and infusing them into patients with tumors to enhance the cellular immune function and improve the anti-tumor effect in the patients. Several types of cellular immunotherapy are currently under study, including chimeric antigen receptor-modified T cell (CAR-T), T cell receptor gene-modified T cell (TCR-T), dendritic cell (DC) vaccine, natural killer cell (NK), tumor- infiltrating lymphocyte (TIL), cytokine-induced killer cell (CIK), and others. While CAR-T cell therapy has developed rapidly in recent years, there are still many deficiencies and challenges in clinical applications, such as varying degrees of neurotoxicity and the risk of cytokine storms, and low efficacy in the treatment of solid tumors. Additionally, most CAR-T cell immunotherapies require autologous adoptive cell transplantation, as allogeneic T cells may lead to graft-versus-host disease (GVHD) unless the HLA barrier is addressed. Moreover, CAR-T cell immunotherapy may cause some side effects that could jeopardize the life of the patient, such as cytokine release syndrome.

In contrast, NK cell therapy has shown good clinical efficacy in the treatment of hematologic tumors and offers more advantages than CAR-T cell therapy. 1) Unlike T cells, NK cells do not lead to a graft-versus-host response (GVHD) and are expected to be a universal cell therapy. 2) Matured NK cells have a relatively short life span (about 7-10 days), which reduces the probability of long-term adverse events while effectively killing tumor cells. 3) NK cells are not restricted by antigen- specific major histocompatibility complex (MHC) and have stronger killing ability. 4) NK cell therapy does not secrete inflammatory factors (IL-1, IL-6, etc.) and carries a low risk of cytokine storms and neurotoxicity. 5) Low-affinity CD16 molecules on NK cell surface can bind to IgG antibody complexes on the target cell surface to mediate antibody-dependent cell-mediated cytotoxicity (ADCC) and can also mediate apoptosis via the Fas/FasL pathway. Furthermore, the low level of programmed death receptor 1 (PD-1) secreted by NK cells hardly induces immunosuppression, thus making NK cells have promising application prospects in the treatment of solid tumors.

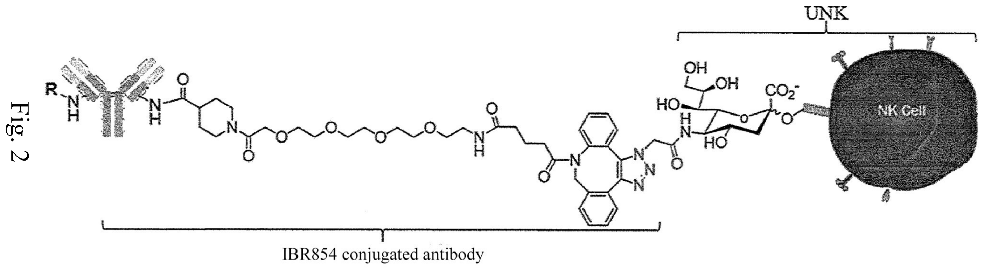

The inventors of the present application have conducted in-depth study in the field of cellular immunotherapy, particularly focusing on the development of an anti-5T4 antibody-natural killer cell conjugate for tumor therapy. FIG. 2 shows a schematic diagram of the structure of an exemplary anti-5T4 antibody-natural killer cell conjugate of the present application.

Natural Killer cells (NK cells) belong to granular lymphocytes and are a component of the human immune system. NK cells recognize target antigens without major histocompatibility complex (MHC) restriction, and allogeneic NK cells carry a very low risk of graft-versus-host disease (GvHD). Therefore, allogeneic NK cell therapy is clinically feasible. In addition, NK cells have a very low risk of causing cytokine release syndrome (CRS). In a Phase I/II clinical trial of CAR-NK cell therapy for lymphoma published in the New England Journal of Medicine in 2020, Liu E et al. demonstrated that 8 out of the 11 enrolled subjects achieved remission after CAR-NK cell infusion, 7 of whom achieved complete remission, and none experienced CRS, neurotoxicity, or GvHD. It can be seen that CAR-NK cell therapy has a high safety. Moreover, it is based on the fact that NK cells recognize target antigens without MHC restriction, NK cells can be prepared into a universal product without being limited by autologous cells, allowing a wide range of NK cell sources for this therapy, such as allogeneic peripheral blood, umbilical cord blood, embryonic stem cells, human-induced pluripotent stem cells, and NK-92 cell lines.

The killing activity of NK cells is primarily mediated by:
1) direct lysis of target cells. NK cells release cytotoxic particles, such as perforin and granzyme, via exocytosis, activating the caspase pathway to induce necrosis or apoptosis of the target cells;
2) secretion of cytokines: cytokine-mediated killing by NK cells which are capable of synthesizing and secreting a variety of cytokines, such as IFN-γ, TNF-α, IL-1, IL-5, IL-8, IL-10, and G-CSF, to induce apoptosis of the target cells;
3) induction of apoptosis. activated NK cells express Fas (CD95) ligand and tumor necrosis factor-related apoptosis-inducing ligand (TRAIL) molecules, inducing apoptosis of CD95+ target cells and TRAIL receptor-positive target cells through a cascade reaction of endogenous enzymes;
4) ADCC: antibody-dependent cell-mediated cytotoxicity;
5) immune checkpoint pathways. NK cells express programmed death receptor 1 (PD-1), cytotoxic T-lymphocyte-associated protein 4 (CTLA4), and the like, and exert their effect by inhibiting the immune checkpoints.

The multiple mechanisms of action described above, as well as the potential and reliable safety of their use as universal products, make NK cell therapy an appealing immunotherapy.

The target antigen selected in the present application is the 5T4 oncofetal trophoblast glycoprotein (referred to as "5T4" in the present application). 5T4 is an N-glycosylated transmembrane protein with a molecular weight of 72 kDa, highly expressed in embryonic trophoblast cells, as well as in various solid tumors (non-small cell lung cancer, breast cancer, pancreatic cancer, gastric cancer, colorectal cancer, ovarian cancer, etc.), and on the surface of tumor stem cells, with low or no expression in normal adult tissues. The extracellular domain of the 5T4 protein contains multiple leucine repeats involved in protein binding and facilitate cell-to-interstitial, i.e., cell-to-cell interactions.

In a first aspect, the present application provides an antibody-natural killer cell (NK cell) conjugate, wherein the antibody is an anti-5T4 antibody or antigen-binding fragment thereof, and the anti-5T4 antibody or antigen-binding fragment thereof is conjugated to the NK cell via a linker.

In some embodiments, the anti-5T4 antibody or antigen-binding fragment thereof comprises:
HCDR1 as set forth in SEQ ID NO: 5 or having at least 80%, 85%, 90%, 95% or 99% identity to the sequence set forth in SEQ ID NO: 5,
HCDR2 as set forth in SEQ ID NO: 6 or having at least 80%, 85%, 90%, 95% or 99% identity to the sequence set forth in SEQ ID NO: 6,
HCDR3 as set forth in SEQ ID NO: 7 or having at least 80%, 85%, 90%, 95% or 99% identity to the sequence set forth in SEQ ID NO: 7,
LCDR1 as set forth in SEQ ID NO: 8 or having at least 80%, 85%, 90%, 95% or 99% identity to the sequence set forth in SEQ ID NO: 8,
LCDR2 as set forth in SEQ ID NO: 9 or having at least 80%, 85%, 90%, 95% or 99% identity to the sequence set forth in SEQ ID NO: 9, and
LCDR3 as set forth in SEQ ID NO: 10 or having at least 80%, 85%, 90%, 95% or 99% identity to the sequence set forth in SEQ ID NO: 10; and
wherein the amino acid sequences of the HCDRs and the LCDRs are defined according to Kabat.

In some embodiments, the amino acid sequence of the heavy chain variable region of the anti-5T4 antibody or antigen-binding fragment thereof is set forth in SEQ ID NO: 1 or has at least 80%, 81%, 82%, 83%, 84%, 85%, 86%, 87%, 88%, 89%, 90%, 91%, 92%, 93%, 94%, 95%, 96%, 97%, 98% or 99% identity to the sequence set forth in SEQ ID NO: 1, and the amino acid sequence of the light chain variable region of the anti-5T4 antibody or antigen-binding fragment thereof is set forth in SEQ ID NO: 3 or has at least 80%, 81%, 82%, 83%, 84%, 85%, 86%, 87%, 88%, 89%, 90%, 91%, 92%, 93%, 94%, 95%, 96%, 97%, 98% or 99% identity to the sequence set forth in SEQ ID NO: 3.

In some embodiments, the amino acid sequence of the heavy chain variable region of the anti-5T4 antibody or antigen-binding fragment thereof differs from the amino acid sequence set forth in SEQ ID NO: 1 by about 1, 2, 3, 4, 5, 6, 7, 8, 9, or 10 amino acid substitutions, deletions, and/or additions. In some embodiments, the C-terminal or N-terminal region of the amino acid sequence set forth in SEQ ID NO: 1 may be truncated by about 1, 2, 3, 4, 5, 6, 7, 8, 9, 10, 11, 12, 13, 14, 15, 20, 25, or more amino acids, while still retaining a function similar to that of the heavy chain variable region of the antibody. In some embodiments, the C-terminal or N-terminal region of the amino acid sequence set forth in SEQ ID NO: 1 may be added with 1, 2, 3, 4, 5, 6, 7, 8, 9, 10, 11, 12, 13, 14, 15, 20, 25, or more amino acids, and the resulting amino acid sequence still retains a function similar to that of the heavy chain variable region of the antibody. In some embodiments, 1, 2, 3, 4, 5, 6, 7, 8, 9, 10, 11, 12, 13, 14, 15, 20, 25, or more amino acids may be added to or deleted from a region other than the C-terminus or the N-terminus of the amino acid sequence set forth in SEQ ID NO: 1, as long as the altered amino acid sequence substantially retains a function similar to that of the heavy chain variable region of the antibody.

In some embodiments, the amino acid sequence of the light chain variable region of the anti-5T4 antibody or antigen-binding fragment thereof differs from the amino acid sequence set forth in SEQ ID NO: 3 by about 1, 2, 3, 4, 5, 6, 7, 8, 9, or 10 amino acid substitutions, deletions, and/or additions. In some embodiments, the C-terminal or N-terminal region of the amino acid sequence set forth in SEQ ID NO: 3 may also be truncated by about 1, 2, 3, 4, 5, 6, 7, 8, 9, 10, 11, 12, 13, 14, 15, 20, 25, or more amino acids, while still retaining a function similar to that of the light chain variable region of the antibody. In some embodiments, 1, 2, 3, 4, 5, 6, 7, 8, 9, 10, 11, 12, 13, 14, 15, 20, 25, or more amino acids may also be added to the C-terminal or N-terminal region of the amino acid sequence set forth in SEQ ID NO: 3, and the resulting amino acid sequence still retains a function similar to that of the light chain variable region of the antibody. In some embodiments, 1, 2, 3, 4, 5, 6, 7, 8, 9, 10, 11, 12, 13, 14, 15, 20, 25, or more amino acids may also be added to or deleted from a region other than the C-terminus or the N-terminus of the amino acid sequence set forth in SEQ ID NO: 3, so long as the altered amino acid sequence substantially retains a function similar to that of the light chain variable region of the antibody.

In some embodiments, the anti-5T4 antibody comprises a heavy chain variable region set forth in SEQ ID NO: 1, a heavy chain constant region set forth in SEQ ID NO: 2, a light chain variable region set forth in SEQ ID NO: 3, and a light chain constant region set forth in SEQ ID NO: 4

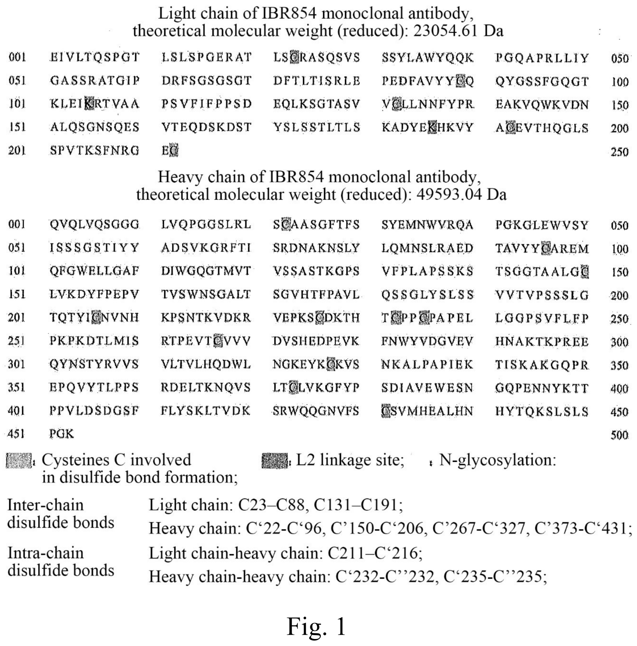

FIG. 1 shows the sequence and structural annotation of an exemplary anti-5T4 antibody of the present application.

In some embodiments, the anti-5T4 antibody is an intact antibody, a single chain fragment variable (scFv) or a bispecific antibody.

In some embodiments, the antigen-binding fragment of the anti-5T4 antibody is a Fab, a Fab', a Fv or a F(ab')₂.

In some embodiments, the anti-5T4 antibody is a humanized antibody or a fully human antibody.

In some embodiments, the anti-5T4 antibody is a monoclonal antibody.

In some embodiments, the anti-5T4 antibody is of IgG1, IgG2 or IgG4 isotype. In some embodiments, the anti-5T4 antibody is of IgG1 isotype.

In some embodiments, the anti-5T4 antibody comprises a light chain constant region of kappa subtype or lambda subtype.

In the case of a given antibody structure/sequence, techniques for preparing the corresponding antibody are well known to those skilled in the art.

As a non-limiting example, the sequence of the anti-5T4 monoclonal antibody can be obtained through phage library screening, which includes screening for binding activity between 5T4 and cells expressing 5T4, followed by confirmation through affinity studies. The anti-5T4 monoclonal antibody is selected and obtained, wherein the anti-5T4 monoclonal antibody comprises the heavy chain variable region set forth in SEQ ID NO: 1 (HCDR1-3 are SEQ ID NOs: 5, 6, and 7, respectively), the heavy chain constant region set forth in SEQ ID NO: 2, the light chain variable region set forth in SEQ ID NO: 3 (LCDR1-3 are SEQ ID NOs: 8, 9, and 10, respectively), and the light chain constant region set forth in SEQ ID NO: 4. The DNA sequence of the anti-5T4 monoclonal antibody is determined, and a recombinant plasmid (IB12) expressing the anti-5T4 monoclonal antibody is constructed. The construction of a monoclonal cell strain is based on known techniques, where the IB12 plasmid are electrotransfected into CHO cells, subjected to one round of minipool screening, followed by two rounds of screening after monoclones are plated, and confirmed by primary culture for passage stability, resulting in a cell strain stably expressing the anti-5T4 monoclonal antibody. The anti-5T4 monoclonal antibody is produced based on known production protocols in the art, including cell culture and protein purification. Working cells are subjected to resuscitation, 3-5 rounds of expansion, and pilot-scale culture, and collected for the unprocessed bulk (UPB) of cells, which undergoes clarification and filtration to generate the cell culture supernatant. The anti-5T4 monoclonal antibody is then obtained by a protein purification process using a three-step chromatography, including Protein A affinity, anion exchange, and cation exchange.

In some embodiments, the NK cell is CD16⁺ and/or NKG2D⁺. In some embodiments, the NK cell is CD16⁺NKG2D⁺.

In some embodiments, the conjugate comprises at least 90%, for example, at least 95%, at least 98%, or at least 99% of CD16⁺NKG2D⁺NK cells. In some embodiments, the conjugate comprises at least 90% of CD16⁺NKG2D⁺NK cells, and at least 95% (e.g., at least 96%, at least 97%, at least 98%, or at least 99%) of CD56⁺NK cells.

There are a variety of techniques for *in vitro* culturing, expanding, and obtaining NK cells, and their general principles and methodologies are known to those skilled in the art.

In some embodiments, NK cells are obtained through *in vitro* culture and expansion of NK cells derived from peripheral blood mononuclear cells (PBMCs), which is also an exemplary method in the examples of the present application. PBMC is one of the main sources of NK cells, offering advantages such as relatively easy collection, ease of *in vitro* expansion, and the absence of toxic and side effects. However, the proportion of NK cells in PBMCs is only 10%-15%, and methods for expanding PBMC-derived NK cells include stimulating their *in vitro* expansion with a combination of cytokines, feeder cells, or membrane particles. These different systems for expansion show varying levels of NK cell expansion efficiency. In some embodiments, HLA and KIR of PBMCs are screened. In some embodiments, variants of CD16a in PBMCs, i.e., 176V, 176F, are screened. In some embodiments, the activity of the natural killer cells during culture is maintained or activated with one or more cytokines. In some embodiments, the proliferation of B cells, macrophages, and other immune cells is inhibited with one or more immunoglobulins or fusion proteins.

PBMCs may be derived from apheresis of peripheral blood lymphocytes from an allogeneic healthy donor. After separating from T cells and red blood cells, the cells are transferred to a primary cell cryopreservation solution and aliquoted according to the specification of ≥ 6.0×10⁷ viable cells per vial to obtain PBMCs. PBMCs are stored at ≤ -175°C in liquid nitrogen for long-term storage. PBMCs frozen in liquid nitrogen are removed, resuscitated in a suitable medium, cultured in an enlarged culture scale (i.e., expanded in an increased volume of culture medium) and added with NK cell-related cytokines which include but are not limited to IL2 and IL15, to maintain their proliferation capacity and activity. Exemplary preparation method can be found in Example 1 of the present application.

In some embodiments, NK cells are obtained through *in vitro* culture and expansion of NK cells derived from umbilical cord blood. There are generally two different methods for obtaining a substantial amount of NK cells from the umbilical cord blood. One method is to expand NK cells in the umbilical cord blood, while the other method is to induce CD34⁺ hematopoietic stem/progenitor cells in the umbilical cord blood to differentiate into NK cells and then expand them.

In some embodiments, NK cells are obtained from *in vitro* culture and expansion of an NK cell line. As an example, NK-92 as a homogeneous immortalized NK lymphoma cell is the first NK cell-based immunotherapy approved by the FDA for clinical trials.

In some embodiments, NK cells are obtained through *in vitro* induction, culture, and expansion of induced pluripotent stem cells (iPSCs) or mesenchymal stem cells (ESCs).

In some embodiments, the anti-5T4 antibody or antigen-binding fragment thereof is conjugated to the NK cell via a click chemical reaction of linkers.

Click chemistry, also known as "link chemistry" or "speed matching combined chemistry", is a concept of synthesis that was introduced by chemist Barry Sharpless in 2001. The main idea is to fast and reliably complete chemical synthesis of various molecules through the splicing of small units. There are four main types of click chemistry reactions: cycloaddition reaction, nucleophilic ring-opening reaction, non-aldol type carbonyl reaction, and carbon-carbon multiple bond addition reaction.

In some embodiments, the anti-5T4 antibody or antigen-binding fragment thereof is conjugated to the NK cell via a first linker conjugated to the anti-5T4 antibody or antigen-binding fragment thereof, and a second linker conjugated to the NK cell, with the first and second linkers conjugated to each other to form the antibody-NK cell conjugate.

In some embodiments, the first linker is an active ester capable of forming a conjugation to a lysine residue of the antibody by a reaction converting an ester bond into an amide bond, for example, the active ester is a pentafluorophenyl ester, such as pentafluorophenyl piperidinate. The active ester can be a molecule that is stable in an aqueous phase and can specifically and covalently bind to the lysine residue of the antibody. In some embodiments, the active ester contains a tetraethylene glycol chain structure that undergoes slow hydrolysis in water. In some embodiments, the reactive ester reacts with an amino group at the hydrophilic interface of the protein, converting the ester bond into an amide bond, thereby establishing a conjugation mechanism.

In some embodiments, the first linker further comprises a carbon-carbon triple bond structure capable of undergoing a cyclization reaction with an azide group to form a five-membered ring of triazole, for example, the carbon-carbon triple bond structure is an octyne group.

In some embodiments, the first linker is a dibenzoazacyclooctyne-glutaryl-amino polyethylene glycol-pentafluorophenyl acetylpiperidinate having the following structure: 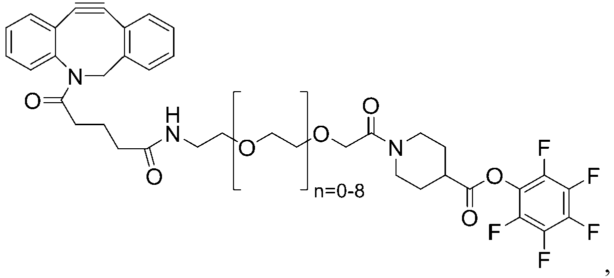 wherein n is an integer of 0-8.

In some embodiments, the first linker is dibenzoazacyclooctyne-glutaryl-amino tetraethylene glycol-pentafluorophenyl acetylpiperidinate having the following structure:

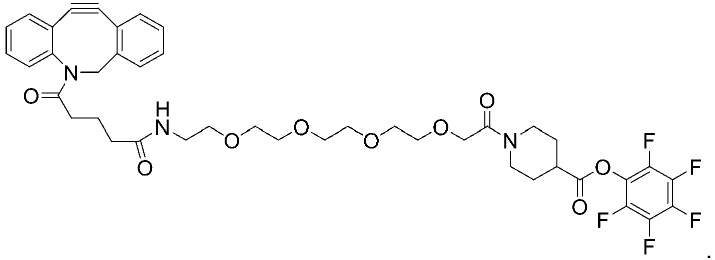

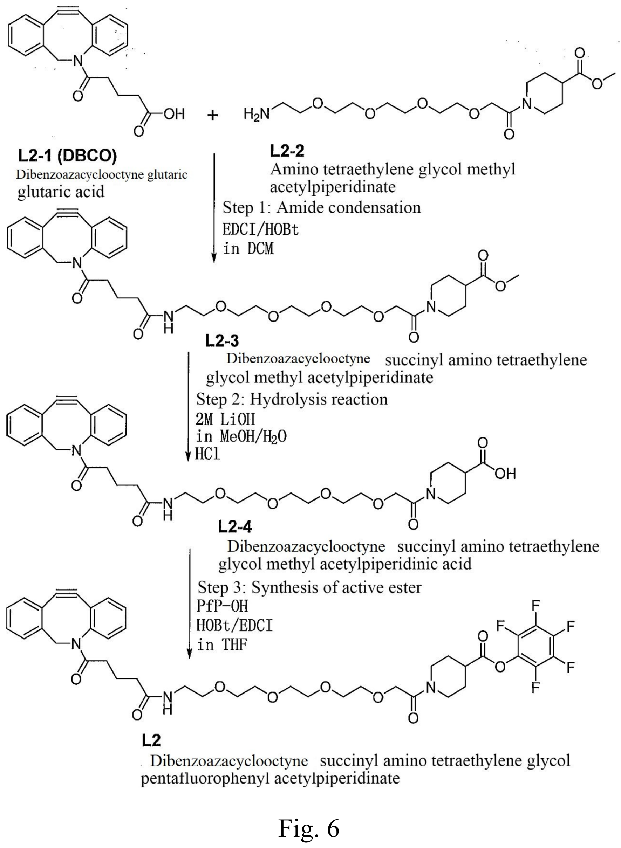

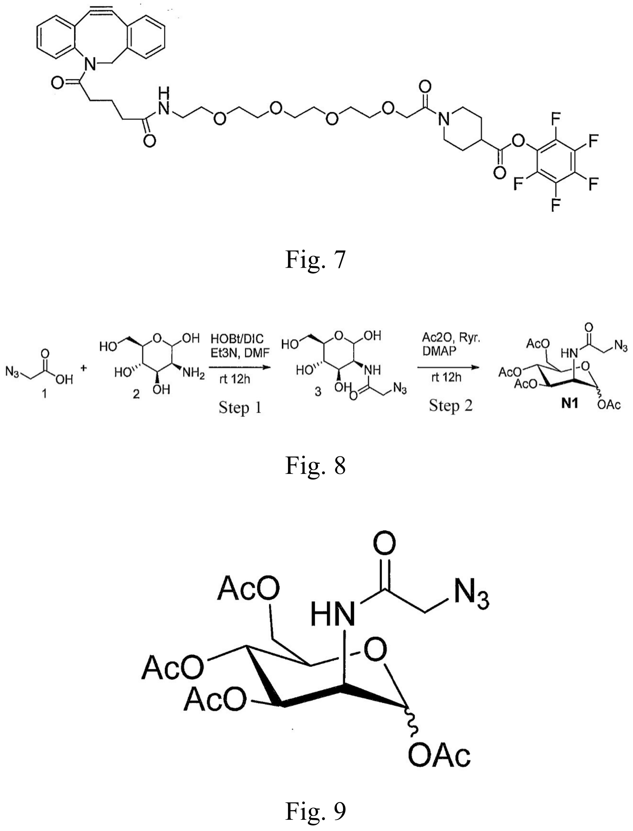

Dibenzoazacyclooctyne-glutaryl-amino tetraethylene glycol-pentafluorophenyl acetylpiperidinate is also referred to as L2 linker in the present application. A process for synthesizing L2 linker is shown in Figure 6, and the structural formula of L2 linker is shown in Figure 7. The process includes three main synthetic steps: amide condensation, hydrolysis reaction, and synthesis of active ester.

### Step 1 - Amide condensation

Dibenzoazacyclooctyne glutaric acid (L2-1) and amino tetraethylene glycol methyl acetylpiperidinate (L2-2) are commercially available. An intermediate dibenzoazacyclooctyne glutaryl amino tetraethylene glycol methyl acetylpiperidinate (L2-3) is obtained according to the chemical condensation reaction. L2-3 is a stable compound.

Specifically, compound L2-1 and compound L2-2 (1:1.1, with L2-2 in excess) are dissolved in a dichloromethane (DCM) solution. Hydroxybenzotriazole (HOBt) and 1-ethyl-(3-dimethylaminopropyl)carbodiimide (EDCI) are separately added, followed by the addition of triethanolamine (TEA). The mixture is stirred at room temperature for 4-12 hours, quenched with water, extracted twice with dichloromethane (DCM), and purified with a silica gel column (dichloromethane: methanol = 20:1) to yield compound L2-3 as a yellow oil.

### Step 2 - Hydrolysis reaction

Intermediate L2-3 obtained in Step 1 is hydrolyzed to yield an intermediate dibenzoazacyclooctyne glutaryl amino tetraethylene glycol acetylpiperidinic acid (L2-4). The hydrolysis reaction is typically a quantitative reaction, and the product is used directly in the next synthesis step without further purification.

Specifically, compound L2-3 is dissolved in a mixed solution of methanol and water, cooled to 0°C, and then added with an aqueous solution of 1 mol/L lithium hydroxide (LiOH). The mixture is stirred for 4-12 hours as the temperature increases from 0°C to room temperature, acidified to pH between 2 and 3 with 1 mol/L HCl, extracted three times with ethyl acetate (EA) and dried to yield the intermediate dibenzoazacyclooctyne glutaryl amino tetraethylene glycol acetylpiperidinic acid (L2-4) as a yellow oil.

### Step 3 - Synthesis of active ester

Intermediate L2-4 obtained in Step 2 (without purification) is condensed with pentafluorophenol and dicyclohexylcarbodiimide (DCC) to yield L2, i.e., dibenzoazacyclooctyne-glutaryl-amino tetraethylene glycol-pentafluorophenyl acetylpiperidinate.

Specifically, compound L2-4 is dissolved in tetrahydrofuran (THF), cooled to 0°C, and then added with pentafluorophenol, hydroxybenzotriazole (HOBt), and dicyclohexylcarbodiimide (DCC). The mixture is stirred for 4-12 hours with temperature increasing from 0°C to room temperature, extracted three times with ethyl acetate (EA), dried and purified with a silica gel column (dichloromethane: methanol = 20:1) to yield dibenzoazacyclooctyne-glutaryl-amino tetraethylene glycol-pentafluorophenyl acetylpiperidinate (L2) as a yellow oil, the structural formula of which is shown in FIG. 7.

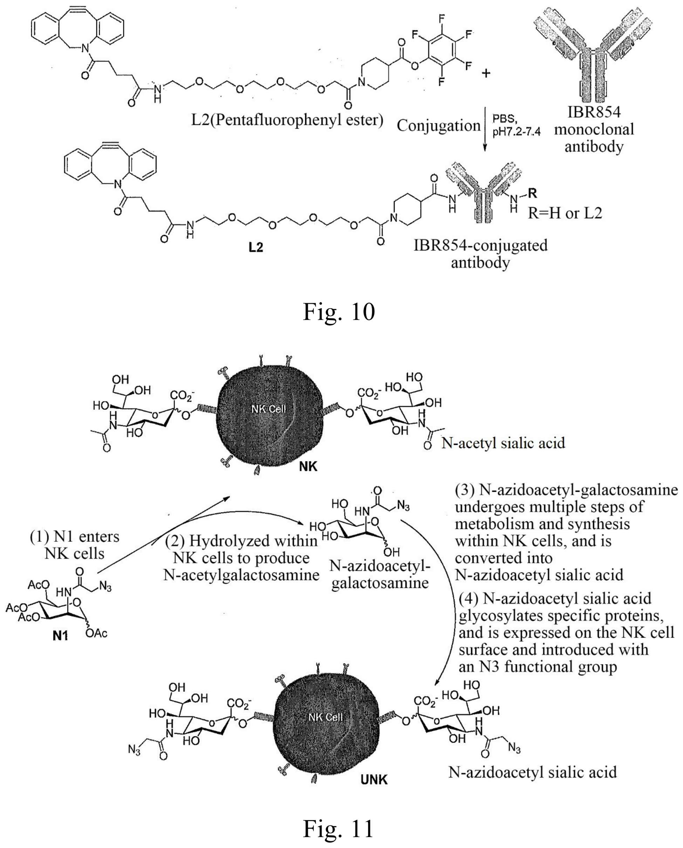

Subsequently, L2 linker may be subjected to a site-directed coupling reaction with the anti-5T4 monoclonal antibody in a system of PBS at pH 7.2-7.4, preferably HEPES at pH 7.2 (see FIG. 10 for a schematic illustration). Typically, one anti-5T4 monoclonal antibody can be conjugated to 1-4 (preferably 1-2) L2 linkers. The reaction can be terminated by adjusting the pH to about 5.0 to yield a L2-conjugated antibody.

In some embodiments, the second linker is azidoacetylcyclohexosamine, such as azidoacetylcyclogalactosamine or azidoacetylglucosamine. In some embodiments, the second linker is a molecule that is stable in an aqueous phase and can specifically and covalently bind to a membrane protein modified by sialic acid. In some embodiments, the second linker is transferred to the membrane protein modified by sialic acid via metabolic pathways in the cell itself during the cell culture. In some embodiments, the azidoacetyl group of the second linker can undergo a cyclization reaction with the carbon-carbon triple bond of the first linker to form a stable five-membered ring of triazole.

In some embodiments, the second linker is 1,3,4,6-oxo-tetraacetyl-2-azidoacetamide-2-deoxy-a,b-D-galactose having the following structure:

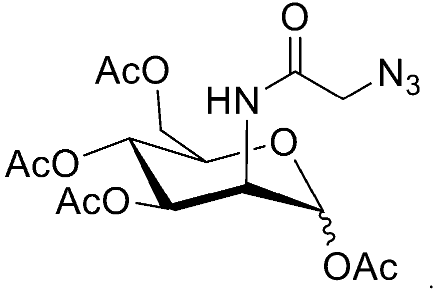

In some embodiments, the second linker comprises at least 90%, for example, at least 95%, at least 98%, or at least 99% of the structure having a single α or β configuration.

1,3,4,6-oxo-tetraacetyl-2-azidoacetamide-2-deoxy-a,b-D-galactose is also referred to as N1 linker in the present application. A process for synthesizing N1 linker is shown in FIG. 8, and the structural formula of N1 linker is shown in FIG. 9. The process includes two main synthetic steps: amino azidoacetylation and hydroxyacetylation.

### Step 1 - Amino azidoacetylation

1.2 equivalents of a-azidoacetic acid (compound 1), 2 equivalents of hydroxybenzotriazole (HOBt), and triethylamine (Et₃N) are added to a solution of D-galactosamine hydrochloride (compound 2) in N,N-dimethylformamide (DMF). A small amount of methanol (MeOH) is added to assist dissolution. The reaction is performed at room temperature for 12 hours. The reaction mixture is poured into dichloromethane (DCM)/methanol (MeOH) and mixed by shaking. An ether is added to precipitate the oily product (compound 3), and the ether layer is decanted. This process is repeated twice, followed by vacuum drying. The product is used in the next reaction without further purification.

### Step 2 - Hydroxyacetylation

Compound 3 obtained above is added to anhydrous pyridine (Pyr.) and acetic anhydride (Ac₂O), followed by the addition of 4-dimethylaminopyridine (DMAP) catalyst at room temperature. The reaction is allowed to proceed for 12 hours. When the reaction is nearly complete, as detected by HPLC, the mixture is concentrated to obtain a racemic mixture of tetraacetyl-N-azidoacetyl-a,b-D-galactosamine. Ethyl acetate/petroleum ether is used to precipitate the product as a solid, mainly in a b-configuration, with a purity of > 85%. Further purification is performed with a silica gel column (dichloromethane: methanol = 20:1), resulting in the N1 product with a single optical isomer purity greater than 90%. Two a,b-isomers can be isomerized to each other in cells, converted to N-azidoacetyl sialic acid through multiple steps of metabolism andsynthesis, and finally expressed on glycoproteins of the NK cell surface. The structural formula of N1 linker is shown in FIG. 9.

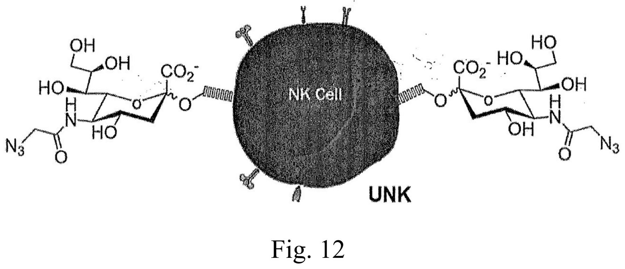

Once NK cells are cultured for 15-16 days, the culture medium is added with N1 linker and incubated for 12-18 hours to obtain N1 linker-modified NK cells (referred to as "UNK" in the examples of the present application) (see Figures 11 and 12 for a schematic diagram of the conjugation mechanism of N1 linker to NK cells and the structure of UNK).

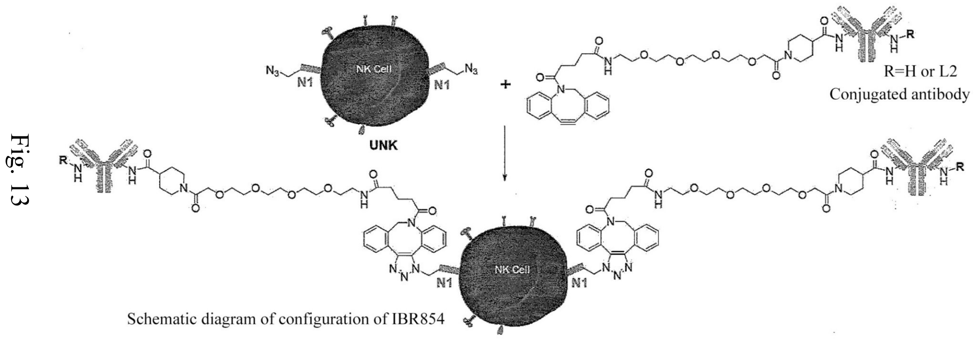

At last, in order to obtain the antibody-NK cell conjugate, the UNK and the L2-conjugated antibody can be subjected to a coupling reaction in the culture medium (the reaction mechanism is shown in FIG. 13) to obtain the antibody-NK cell conjugate.

Further, subsequent processes include the preparation of an antibody-NK cell conjugate formulation to effectively prolong the stability of the antibody-NK cell conjugate. The formulation may contain isotonic-maintaining components such as sodium chloride and human serum albumin, and may also contain components such as trehalose, sucrose, dextran, DMSO, and the like, which maintain the cells' tolerance to low temperature and the activity of proteins and enzymes.

In a second aspect, the present application provides a cell population comprising the antibody-natural killer cell (NK cell) conjugate of the first aspect.

In some embodiments, the count of CD3⁻CD56⁺CD16⁺ cells is at least 95% with respect to the total cell count in the cell population. In some embodiments, the count of CD3⁻CD56⁺CD16⁺ cells is at least 98% with respect to the total cell count in the cell population. In some embodiments, the count of CD3⁻CD56⁺NKG2D⁺ cells is at least 95% with respect to the total cell count in the cell population. In some embodiments, the count of CD3⁻CD56⁺NKG2D⁺ cells is at least 98% with respect to the total cell count in the cell population.

In some embodiments, the count of CD3⁺CD56⁺ cells is no more than 5% with respect to the total cell count in the cell population.

In some embodiments, the count of CD3-CD19⁺ cells is no more than 2% with respect to the total cell count in the cell population.

In some embodiments, the counts of CD3⁺CD4⁺ and CD3⁺CD8⁺ cells are no more than 2% with respect to the total cell count in the cell population.

In some embodiments, by cell count, the antibody-natural killer cell (NK cell) conjugate is at least 90% with respect to the total cell count in the cell population. In some embodiments, by cell count, the antibody-natural killer cell (NK cell) conjugate is at least 95% with respect to the total cell count in the cell population. In some embodiments, by cell count, the antibody-natural killer cell (NK cell) conjugate is at least 98% with respect to the total cell count in the cell population. In some embodiments, by cell count, the antibody-natural killer cell (NK cell) conjugate is at least 99% with respect to the total cell count in the cell population.

In a third aspect, the present application provides a pharmaceutical composition comprising the antibody-natural killer cell (NK cell) conjugate of the first aspect or the cell population of the second aspect, and a pharmaceutically acceptable carrier.

In some embodiments, the pharmaceutical composition comprises sodium chloride and/or human serum albumin.

In some embodiments, the pharmaceutical composition comprises trehalose, sucrose, dextran, DMSO, or any combination thereof.

In some embodiments, the pharmaceutical composition is for use in the treatment of a tumor in an individual.

In some embodiments, the tumor is a tumor with a high expression of 5T4 (5T4⁺). In some embodiments, a tumor with a high expression of 5T4 (5T4⁺) refers to a tumor in which at least 60% of the tumor cells in the tumor cell population express 5T4. In some embodiments, a tumor with a high expression of 5T4 (ST4⁺) refers to a tumor in which at least 70% of the tumor cells in the tumor cell population express 5T4. In some embodiments, a tumor with a high expression of 5T4 (ST4⁺) refers to a tumor in which at least 80% of the tumor cells in the tumor cell population express 5T4. In some embodiments, a tumor with a high expression of 5T4 (5T4⁺) refers to a tumor in which at least 90% of the tumor cells in the tumor cell population express 5T4. In some embodiments, a tumor with a high expression of 5T4 (ST4⁺) refers to a tumor in which at least 95% of the tumor cells in the tumor cell population express 5T4. In some embodiments, a tumor with a high expression of 5T4 (5T4⁺) refers to a tumor in which at least 98% of the tumor cells in the tumor cell population express 5T4. In some embodiments, a tumor with a high expression of 5T4 (5T4⁺) refers to a tumor in which at least 99% of the tumor cells in the tumor cell population express 5T4.

In some embodiments, the tumor is a solid tumor.

In some embodiments, the tumor is a hematological tumor.

In some embodiments, the tumor is a malignant tumor.

In some embodiments, the tumor is a cancer.

In some embodiments, the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, ovarian cancer, renal cancer, bladder cancer, cervical cancer, colon cancer, liver cancer, prostate cancer, peritoneal cancer, hepatocellular cancer, glioblastoma, urinary tract cancer, rectal cancer, endometrial or uterine cancer, salivary gland cancer, squamous cell cancer (e.g., squamous cell carcinoma), vulvar cancer, thyroid cancer, anal cancer, penile cancer, melanoma, B-cell lymphoma, brain cancer, head and neck cancer, and metastases of the above cancers.

In some embodiments, the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, and ovarian cancer.

In some embodiments, the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, and colorectal cancer.

In a fourth aspect, the present application provides the use of the antibody-natural killer cell (NK cell) conjugate of the first aspect or the cell population of the second aspect in the manufacture of a medicament for the treatment of a tumor in an individual.

In some embodiments, the tumor is a tumor with a high expression of 5T4 (5T4⁺). In some embodiments, a tumor with a high expression of 5T4 (5T4⁺) refers to a tumor in which at least 60% of the tumor cells in the tumor cell population express 5T4. In some embodiments, a tumor with a high expression of 5T4 (ST4⁺) refers to a tumor in which at least 70% of the tumor cells in the tumor cell population express 5T4. In some embodiments, a tumor with a high expression of 5T4 (5T4⁺) refers to a tumor in which at least 80% of the tumor cells in the tumor cell population express 5T4. In some embodiments, a tumor with a high expression of 5T4 (ST4⁺) refers to a tumor in which at least 90% of the tumor cells in the tumor cell population express 5T4. In some embodiments, a tumor with a high expression of 5T4 (5T4⁺) refers to a tumor in which at least 95% of the tumor cells in the tumor cell population express 5T4. In some embodiments, a tumor with a high expression of 5T4 (ST4⁺) refers to a tumor in which at least 98% of the tumor cells in the tumor cell population express 5T4. In some embodiments, a tumor with a high expression of 5T4 (5T4⁺) refers to a tumor in which at least 99% of the tumor cells in the tumor cell population express 5T4.

In some embodiments, the tumor is a solid tumor.

In some embodiments, the tumor is a hematological tumor.

In some embodiments, the tumor is a malignant tumor.

In some embodiments, the tumor is a cancer.

In some embodiments, the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, ovarian cancer, renal cancer, bladder cancer, cervical cancer, colon cancer, liver cancer, prostate cancer, peritoneal cancer, hepatocellular cancer, glioblastoma, urinary tract cancer, rectal cancer, endometrial or uterine cancer, salivary gland cancer, squamous cell cancer (e.g., squamous cell carcinoma), vulvar cancer, thyroid cancer, anal cancer, penile cancer, melanoma, B-cell lymphoma, brain cancer, head and neck cancer, and metastases of the above cancers.

In some embodiments, the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, and ovarian cancer.

In some embodiments, the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, and colorectal cancer.

In a fifth aspect, the present application provides a method of treating a tumor in an individual, the method comprising administering to the individual an effective amount of the antibody-natural killer cell (NK cell) conjugate of the first aspect, or the cell population of the second aspect, or the pharmaceutical composition of the third aspect.

In some embodiments, the tumor is a tumor with a high expression of 5T4 (5T4⁺). In some embodiments, a tumor with a high expression of 5T4 (5T4⁺) refers to a tumor in which at least 60% of the tumor cells in the tumor cell population express 5T4. In some embodiments, a tumor with a high expression of 5T4 (ST4⁺) refers to a tumor in which at least 70% of the tumor cells in the tumor cell population express 5T4. In some embodiments, a tumor with a high expression of 5T4 (5T4⁺) refers to a tumor in which at least 80% of the tumor cells in the tumor cell population express 5T4. In some embodiments, a tumor with a high expression of 5T4 (ST4⁺) refers to a tumor in which at least 90% of the tumor cells in the tumor cell population express 5T4. In some embodiments, a tumor with a high expression of 5T4 (5T4⁺) refers to a tumor in which at least 95% of the tumor cells in the tumor cell population express 5T4. In some embodiments, a tumor with a high expression of 5T4 (ST4⁺) refers to a tumor in which at least 98% of the tumor cells in the tumor cell population express 5T4. In some embodiments, a tumor with a high expression of 5T4 (5T4⁺) refers to a tumor in which at least 99% of the tumor cells in the tumor cell population express 5T4.

In some embodiments, the tumor is a solid tumor.

In some embodiments, the tumor is a hematological tumor.

In some embodiments, the tumor is a malignant tumor.

In some embodiments, the tumor is a cancer.

In some embodiments, the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, ovarian cancer, renal cancer, bladder cancer, cervical cancer, colon cancer, liver cancer, prostate cancer, peritoneal cancer, hepatocellular cancer, glioblastoma, urinary tract cancer, rectal cancer, endometrial or uterine cancer, salivary gland cancer, squamous cell cancer (e.g., squamous cell carcinoma), vulvar cancer, thyroid cancer, anal cancer, penile cancer, melanoma, B-cell lymphoma, brain cancer, head and neck cancer, and metastases of the above cancers.

In some embodiments, the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, and ovarian cancer.

In some embodiments, the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, and colorectal cancer.

It is to be understood that the foregoing detailed description is intended only to enable those skilled in the art to have a better understanding of the present application and is not intended to limit in any way. Various modifications and variations can be made to the described embodiments by those skilled in the art.

### Examples

The present application is further illustrated with reference to specific examples. It should be understood that these examples are provided for illustrative purposes only and are not intended to limit the scope of the present application.

### Example 1 - Preparation of anti-5T4 antibody-NK cell conjugate

The preparation of the antibody-NK cell conjugate in this example can generally be divided into the following steps:
(1) preparation of the anti-5T4 monoclonal antibody;
(2) preparation of the NK cell;
(3) preparation of the antibody linker (referred to as L2 linker in this example);
(4) preparation of the NK cell linker (referred to as N1 linker in this example);
(5) applying the antibody linker (L2 linker) to the antibody;
(6) applying the NK cell linker (N1 linker) to the NK cell; and
(7) conjugating the antibody to the NK cell, each carrying their respective linkers.

### (1) Preparation of anti-5T4 monoclonal antibody

Briefly, the sequence of the anti-5T4 monoclonal antibody was obtained through phage library screening, which included screening for binding activity between 5T4 and cells expressing 5T4, followed by confirmation through affinity studies. The anti-5T4 monoclonal antibody was selected and obtained, wherein the anti-5T4 monoclonal antibody comprises the heavy chain variable region set forth in SEQ ID NO: 1 (HCDR1-3 are SEQ ID NOs: 5, 6, and 7, respectively), the heavy chain constant region set forth in SEQ ID NO: 2, the light chain variable region set forth in SEQ ID NO: 3 (LCDR1-3 are SEQ ID NOs: 8, 9, and 10, respectively), and the light chain constant region set forth in SEQ ID NO: 4. The DNA sequence of the anti-5T4 monoclonal antibody was determined, and a recombinant plasmid (IB12) expressing the anti-5T4 monoclonal antibody was constructed.

The construction of a monoclonal cell strain was based on known techniques, where the IB12 plasmid was electrotransfected into CHO cells, subjected to one round of minipool screening, followed by two rounds of screening after monoclones were plated, and confirmed by primary culture for passage stability, resulting in a cell strain stably expressing the anti-5T4 monoclonal antibody.

The anti-5T4 monoclonal antibody was produced based on known production protocols in the art, including cell culture and protein purification. Working cells were subjected to resuscitation, 3-5 rounds of expansion, and pilot-scale culture, and collected for the unprocessed bulk (UPB) of cells, which underwent clarification and filtration to generate the cell culture supernatant. The anti-5T4 monoclonal antibody was then obtained by a protein purification process using a three-step chromatography, including Protein A affinity, anion exchange, and cation exchange.

### (2) Preparation of NK cell

PBMCs were derived from apheresis of peripheral blood lymphocytes from an allogeneic healthy donor. After separating T cells and red blood cells, the cells were transferred to a primary cell cryopreservation solution and aliquoted according to the specification of ≥6.0×10⁷ viable cells per vial to obtain PBMCs. PBMCs were stored at ≤-175°C in liquid nitrogen for long-term storage.

PBMCs frozen in liquid nitrogen were removed, resuscitated in a suitable medium, cultured in an enlarged culture scale (i.e., expanded in an increased volume of culture medium) and added with NK cell-related cytokines which included but were not limited to IL2 and IL15, to maintain their proliferation capacity and activity. Exemplary NK cell culture stages, process parameters, and process control indicators are shown in Table 1 below.

**Table 1**

| Culture stage | Process parameters | Process control |
|---|---|---|
| Days 0-5 | 1. Temperature of resuscitation: 37°C | 1.PBMC resuscitation |
| Primary culture of NK cells | | Seeding density of viable cells: (1.00~2.00) ×10⁶ cells/mL |
| | Serum: 10% | |
| | Culture system: 120±15 mL | |
| | | Cell viability: ≥80.0% |
| | Coating: incubation with coating factor combination at room temperature for at least 1 h or 2-8°C coating overnight. | 2. Day 5: Cell viability ≥60.0% |
| | 2. Culture conditions: Temperature of 37±0.5°C, CO₂ concentration of 5±0.5% | |
| | 3. Fluid supplementation Day 3: 54 mL NK cell activation medium and 6 mL serum (serum volume was 10% of the total volume of fluid supplementation) were added. | |
| Days 5-15 | 1. Fluid supplementation: | 1. Days 5, 7, and 9: density after the supplementation: ≥3.00×10⁵ cells/mL |
| Expansion culture of NK cells | Day 5: After the cell suspension was homogeneously placed in a culture bag, NK cell activation medium and serum were added, with a total volume of the fluid supplementation of 100-420 mL (the volume of serum accounted for 10% of the total volume of the fluid supplementation). | |
| | | 2. Day 9: Cell viability ≥70.0% Value of NK purity (CD3⁻ CD56⁺) was reported Value of T-cell proportion (CD3⁺CD56⁻) was reported |
| | Day 7: 10-30 mL serum was added, and NK cell expansion medium (containing 0.5% human albumin) was supplemented, with a total volume of the fluid supplementation of 100-700 mL. | 3. Days 13-15: Density after the supplementation: ≥6.00×10⁵ cells/mL Cell viability: ≥ 75.0% NK purity (CD3⁻CD56⁺) ≥85.0% |
| | | NKT cell proportion (CD3⁺CD56⁺) ≤4.0% Sterility test |
| | Day 9: Cell suspension was added at 1:3 into three cell culture bags, each supplemented with 200- | |
| | 800 mL NK cell expansion medium (containing 0.5% human albumin). | |
| | Day 11: 100-800 mL NK cell expansion medium (containing 0.5% human albumin) | |
| | Day 13-15: Each bag of cell suspension was split 1:2 into two cell culture bags, followed by addition of NK cell expansion medium. (containing 0.5% human albumin) | |
| | 2. Culture conditions: temperature of 37±0.5°C, CO₂ concentration of 5±0.5% | |

The quality control for the purity of final NK cells fulfilled the requirements specified in Table 2 below (other parameters, such as cell density and biosafety, complied with industry standards):

**Table 2.**

| Cell type | | Detection method | Standard |
|---|---|---|---|
| NK cell | NK: CD3⁻CD56⁺ | Flow cytometry | ≥95.0% |
| | NKT: CD3⁺CD56⁺ | | ≤5.0% |
| | CD3⁻CD56⁺CD16⁺ | | ≥95.0% |
| | CD3⁻CD56⁺NKG2D⁺ | | ≥95.0% |
| B cell (CD3⁻CD19⁺) | | | ≤2.0% |
| T cell (CD3⁺CD4⁺&CD3⁺CD8⁺) | | | ≤2.0% |

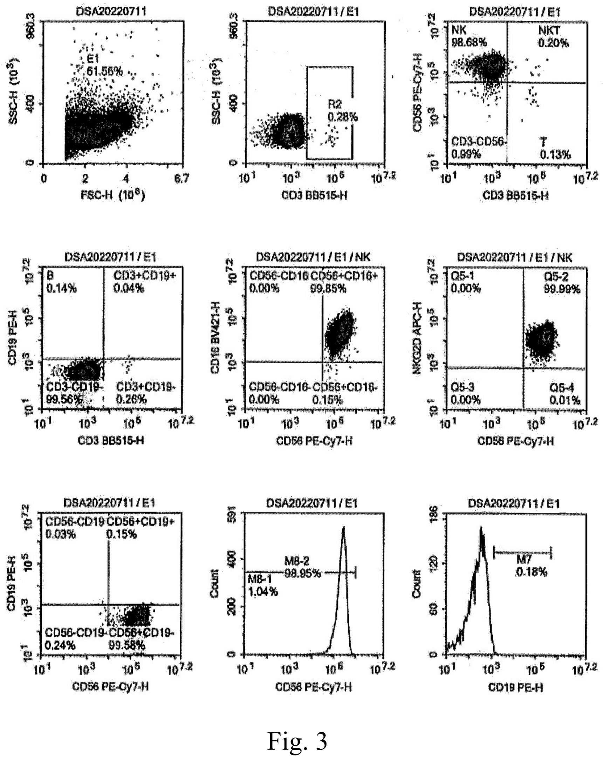

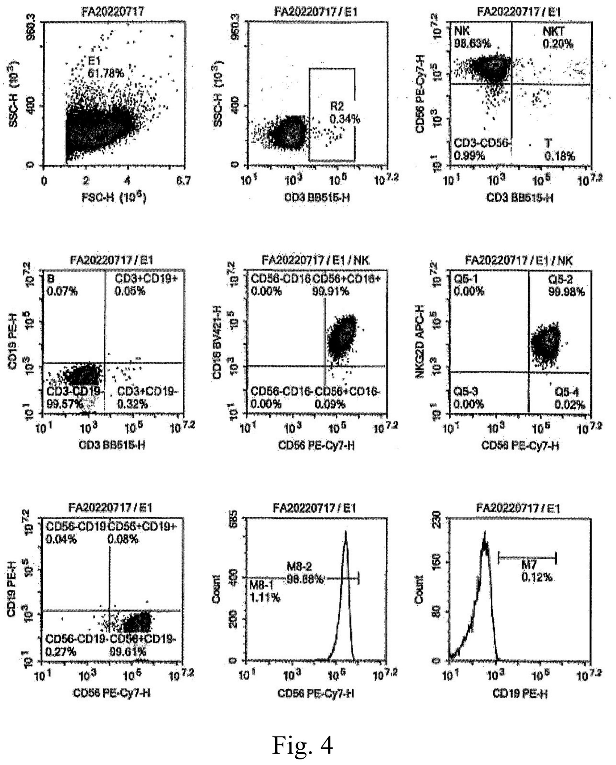

Figures 3 and 4 show results of flow cytometric detection of the immunological markers described above in the final prepared stock solution and the formulation of antibody-NK cell conjugates.

### (3) Preparation of antibody linker (referred to as L2 linker in this example)

The process for synthesizing L2 linker is shown in Figure 6, and the structural formula of L2 linker is shown in Figure 7. The process included three main synthetic steps: amide condensation, hydrolysis reaction, and synthesis of active ester.

### Step 1 - Amide condensation

Dibenzoazacyclooctyne glutaric acid (L2-1) and amino tetraethylene glycol methyl acetylpiperidinate (L2-2) were commercially available. An intermediate dibenzoazacyclooctyne glutaryl amino tetraethylene glycol methyl acetylpiperidinate (L2-3) was obtained according to the chemical condensation reaction. L2-3 is a stable compound.

Specifically, compound L2-1 and compound L2-2 (1:1.1, with L2-2 in excess) were dissolved in a dichloromethane (DCM) solution. Hydroxybenzotriazole (HOBt) and 1-ethyl-(3-dimethylaminopropyl)carbodiimide (EDCI) were separately added, followed by the addition of triethanolamine (TEA). The mixture was stirred at room temperature for 4-12 hours, quenched with water, extracted twice with dichloromethane (DCM), and purified with a silica gel column (dichloromethane: methanol = 20:1) to yield compound L2-3 as a yellow oil.

### Step 2 - Hydrolysis reaction

Intermediate L2-3 obtained in Step 1 was hydrolyzed to yield an intermediate dibenzoazacyclooctyne glutaryl amino tetraethylene glycol acetylpiperidinic acid (L2-4). The hydrolysis reaction was typically a quantitative reaction, and the product was used directly in the next synthesis step without further purification.

Specifically, compound L2-3 was dissolved in a mixed solution of methanol and water, cooled to 0°C, and then added with an aqueous solution of 1 mol/L lithium hydroxide (LiOH). The mixture was stirred for 4-12 hours with temperature increasing from 0°C to room temperature, acidified to pH between 2 and 3 with 1 mol/L HCl, extracted three times with ethyl acetate (EA) and dried to yield the intermediate dibenzoazacyclooctyne glutaryl amino tetraethylene glycol acetylpiperidinic acid (L2-4) as a yellow oil.

### Step 3 - Synthesis of active ester

Intermediate L2-4 obtained in Step 2 (without purification) was condensed with pentafluorophenol and dicyclohexylcarbodiimide (DCC) to yield L2, i.e., dibenzoazacyclooctyne-glutaryl-amino tetraethylene glycol-pentafluorophenyl acetylpiperidinate.

Specifically, compound L2-4 was dissolved in tetrahydrofuran (THF), cooled to 0°C, and then added with pentafluorophenol, hydroxybenzotriazole (HOBt), and dicyclohexylcarbodiimide (DCC). The mixture was stirred for 4-12 hours with temperature increasing from 0°C to room temperature, extracted three times with ethyl acetate (EA), dried and purified with a silica gel column (dichloromethane: methanol = 20:1) to yield dibenzoazacyclooctyne-glutaryl-amino tetraethylene glycol-pentafluorophenyl acetylpiperidinate (L2) as a yellow oil, the structural formula of which is shown in FIG. 7.

### (4) Preparation of NK cell linker (referred to as N1 linker in this example)

The process for synthesizing N1 linker is shown in FIG. 8, and the structural formula of N1 linker is shown in FIG. 9. The process included two main synthetic steps: amino azidoacetylation and hydroxyacetylation.

### Step 1 - Amino azidoacetylation

1.2 equivalents of a-azidoacetic acid (compound 1), 2 equivalents of hydroxybenzotriazole (HOBt), and triethylamine (Et₃N) were added to a solution of D-galactosamine hydrochloride (compound 2) in N,N-dimethylformamide (DMF). A small amount of methanol (MeOH) was added to assist dissolution. The reaction was performed at room temperature for 12 hours. The reaction mixture was poured into dichloromethane (DCM)/methanol (MeOH) and mixed by shaking. An ether was added to precipitate the oily product (compound 3), and the ether layer was decanted. This process was repeated twice, followed by vacuum drying. The product was used in the next reaction without further purification.

### Step 2 - Hydroxyacetylation

Compound 3 obtained above was added to anhydrous pyridine (Pyr.) and acetic anhydride (Ac₂O), followed by the addition of 4-dimethylaminopyridine (DMAP) catalyst at room temperature. The reaction was allowed to proceed for 12 hours. When the reaction was nearly complete, as detected by HPLC, the mixture was concentrated to obtain a racemic mixture of tetraacetyl-N-azidoacetyl-a,b-D-galactosamine. Ethyl acetate/petroleum ether was used to precipitate the product as a solid, mainly in a b-configuration, with a purity of >85%. Further purification was performed with a silica gel column (dichloromethane: methanol = 20:1), resulting in the N1 product with a single optical isomer purity greater than 90%. The two a,b- isomers were capable of being isomerized to each other in cells, converted to N-azidoacetyl sialic acid through multiple steps of metabolism and synthesis, and finally expressed on glycoproteins of the NK cell surface. The structural formula of N1 linker is shown in FIG. 9.

### (5) Applying antibody linker (L2 linker) to antibody

L2 linker was capable of generating a site-directed coupling reaction with the anti-5T4 monoclonal antibody in a system of PBS at pH 7.2-7.4, preferably HEPES at pH 7.2 (see FIG. 10 for a schematic illustration). Typically, one anti-5T4 monoclonal antibody was able to be conjugated to 1-4 (preferably 1-2) L2 linkers. The reaction was capable of being terminated by adjusting the pH to about 5.0 to yield a L2-conjugated antibody.

### (6) Applying NK cell linker (N1 linker) to NK cell

Once NK cells were cultured for 15-16 days, the culture medium was added with N1 linker and incubated for 12-18 hours to obtain N1 linker-modified NK cells (referred to as "UNK" in the examples of the present application) (see Figures 11 and 12 for a schematic diagram of the conjugation mechanism of N1 linker to NK cells and the structure of UNK).

### (7) Conjugating the antibody to the NK cell, each carrying their respective linkers

The UNK and the L2-conjugated antibody were subjected to a coupling reaction in the culture medium (see Figure 13 for the reaction mechanism) to obtain a stock solution (DS) of the antibody-NK cell conjugate. The anti-5T4 monoclonal antibody-NK cell conjugate was designated as "IBR854", which was used in the subsequent examples to refer to the anti-5T4 antibody-NK cell conjugate.

Further, subsequent processes included the preparation of an antibody-NK cell conjugate formulation to effectively prolong the stability of the antibody-NK cell conjugate. The formulation could contain isotonic-maintaining components such as sodium chloride and human serum albumin, and could also contain components such as trehalose, sucrose, dextran, DMSO, and the like, which maintained the cells' tolerance to low temperature and the activity of proteins and enzymes.

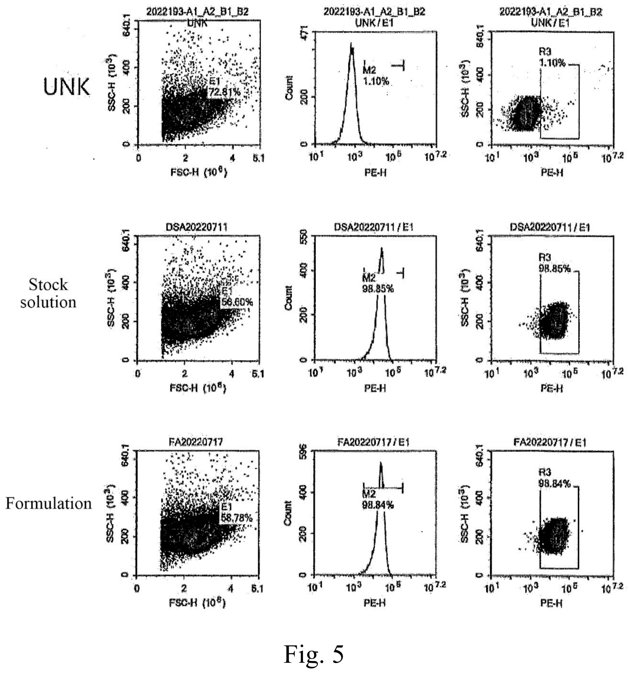

Purities of NK cells in the antibody-NK cell conjugate stock solution (FIG. 3) and the formulation (FIG. 4) were measured by flow cytometry. Results showed values of above 98%, which were larger than 95% as required by standards. In addition, the conjugation positivity rates of the antibody-NK cell conjugate stock solution and the formulation were assessed by flow cytometry, with UNK as a control. Results showed that the conjugation positivity rates of both the stock solution and the formulation were above 98% (FIG. 5), while the standard requirement was generally 90%.

### Example 2 - Comparison of primary antibody properties of conjugate IBR854 and anti-5T4 monoclonal antibody

In this example, conjugate IBR854 prepared in Example 1 was compared in terms of several primary antibody properties with the anti-5T4 monoclonal antibody prepared in Step (1) in Example 1. The results are shown in Table 3 below. Methodology for each test item followed conventional assays in the art.

**Table 3.**

| Test item | | Anti-5T4 monoclonal antibody | IBR854-conjugated antibody |
|---|---|---|---|
| Isoelectric point (by icIEF method) | | Main peak pl, 8.65 | Characteristic peaks 1/2/3, 8.3/8.5/8.6 |
| Stability (by DSF method) | | Tm1**:** 61.6°C; Tm2: 72.2°C | Tm1: 64.8°C; Tm2: 74.2°C |
| Affinity to target (by BLI method) | Human 5T4 | 1.38×10⁻⁸ mol/L | 3.75×10⁻⁸ mol/L |
| Affinity to Fc receptor (by BLI method) | FcγRI | 2.04×10⁻⁹ mol/L | 5.66×10⁻¹⁰ mol/L |
| | FcγRIIa | 1.12×10⁻⁸ mol/L | 8.24×10⁻⁹ mol/L |
| | FcγRIIIa (V176) | 3.33×10⁻⁸ mol/L | 2.32×10⁻⁸ mol/L |
| | FcγRIIIa (F176) | 3.41×10⁻⁷ mol/L | 1.62×10⁻⁷ mol/L |
| | FcRn | 4.43×10⁻⁹ mol/L | 3.44×10⁻⁹ mol/L |
| | C1q | 2.14×10⁻⁹ mol/L | 1.19×10⁻⁹ mol/L |

As can be seen from the results in Table 3, conjugation of the anti-5T4 monoclonal antibody to NK cells did not significantly affect properties of the antibody itself, allowing its desired characteristics to be preserved.

### Example 3 - Evaluation of in vitro killing activity of conjugate IBR854 on lung cancer, breast cancer and pancreatic cancer cells

In the evaluation of cell-killing activity in this example, eight tumor cell lines expressing 5T4 were selected, including lung cancer cell lines H1975, NCI-H292, NCI-H226 and HCC827, breast cancer cell lines MDA-MB-231 and MDA-MB-468, and pancreatic cancer cell lines Panc-1 and BxPC3, 5T4 expression levels of which were pre-determined (by flow cytometry) and as shown in Table 4 below:

**Table 4.**

| **Cell line** | **5T4 expression rate** |
|---|---|
| H1975 | 82.1% |
| NCI- H292 | 68.3% |
| NCI-H226 | 98.1% |
| MDA-MB-231 | 78.0% |
| MDA-MB-468 | 73.5% |
| Panc-1 | 87.7% |
| BxPC3 | 71.7% |
| HCC827 | 82.1% |

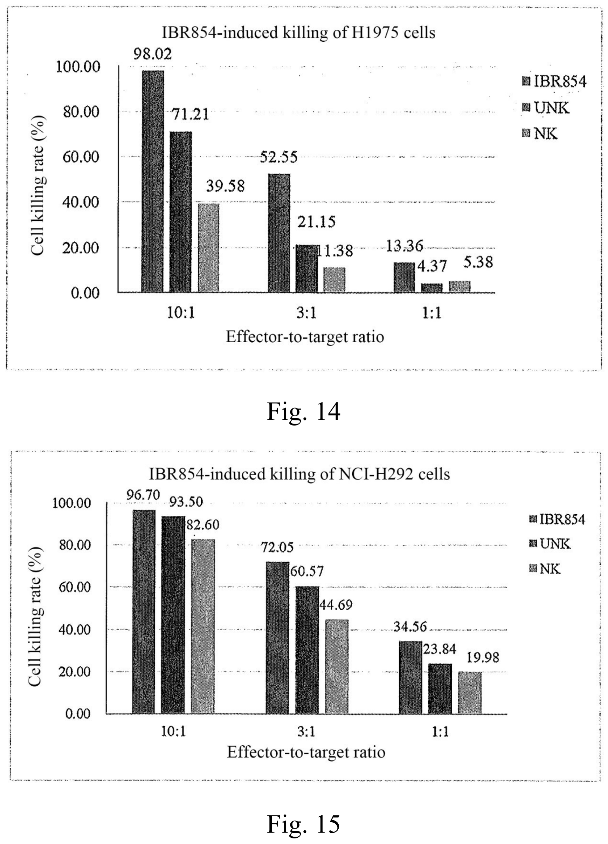

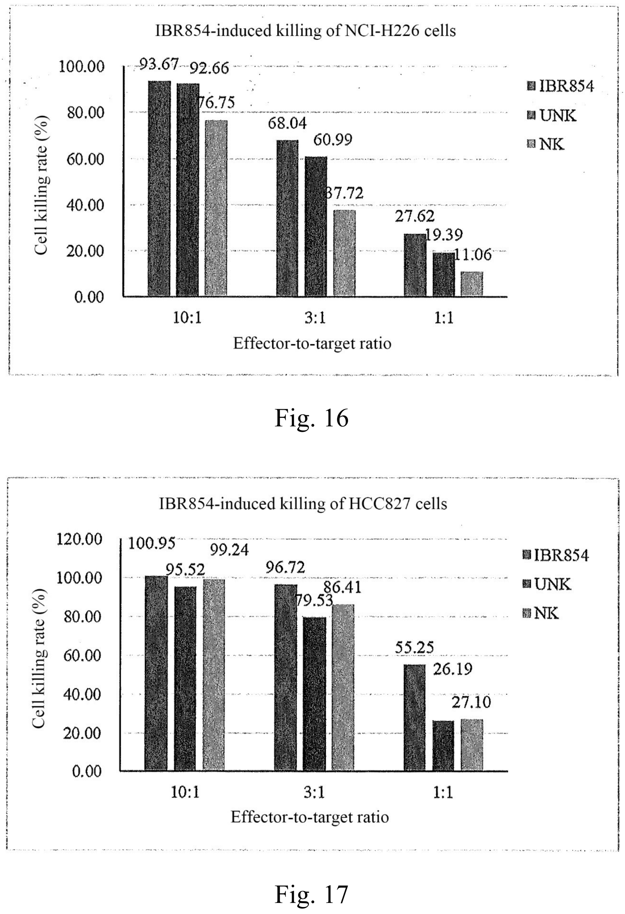

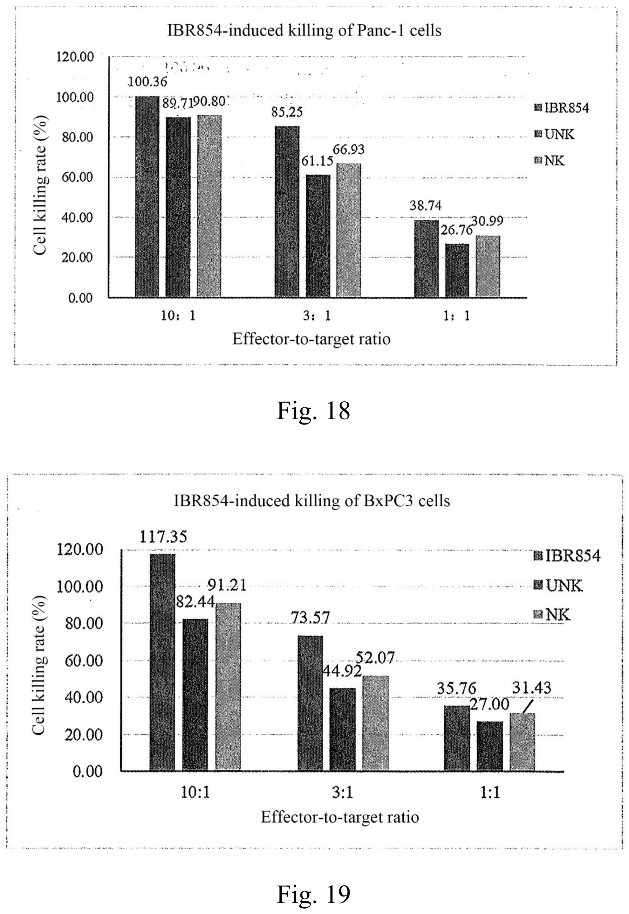

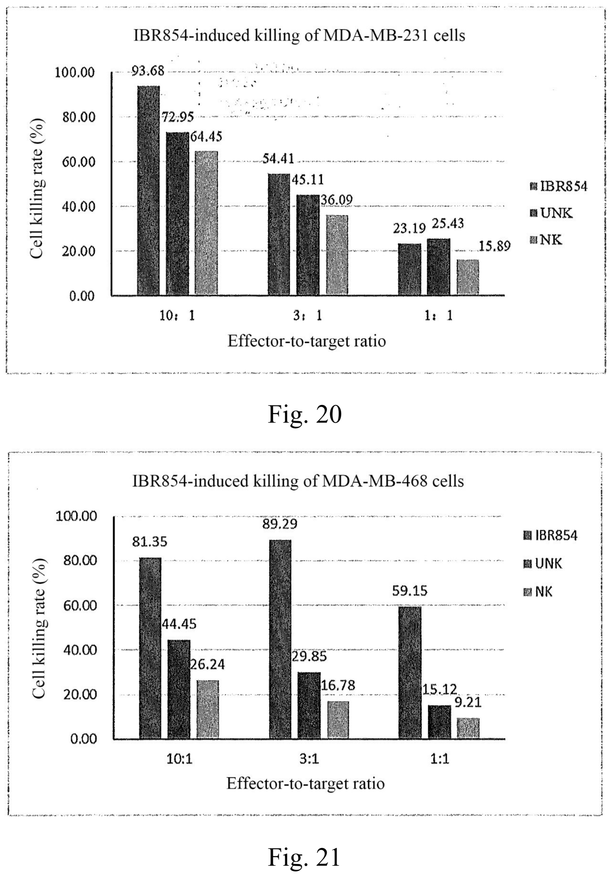

First, the cell-killing activities of conjugate IBR854, as well as the NK cells (obtained in step (2) of Example 1) and the UNK cells (obtained in step (6) of Example 1) as controls, against 8 tumor cell lines were evaluated with a real-time, label-free cellular assay technique. A tumor cell-only control group was included in the experiment. Each of the NK cells, the UNK cells, and conjugate IBR854 were set at three concentrations, with three replicate wells per concentration. Tumor cells were seeded into assay plates, and the NK cells, the UNK cells, and IBR854 were added. Cell index (CI) values were monitored and recorded at 15-minute intervals with an RTCA instrument. Cell-killing rates were calculated based on changes in the cell index. Results are shown in FIG. 14 to FIG. 21. The results show that IBR854 exhibited a killing rate of over 85% against 8 cell lines of lung cancer, breast cancer, and pancreatic cancer, and its killing activity was higher than those of NK cells and UNK cells.

Next, the dose-response relationship in the IBR854 induced killing of the 8 tumor cell lines was measured with the Calcein AM release method. IBR854 was tested at eight concentrations, with six replicate wells per concentration. Changes in fluorescence intensity were detected with a microplate reader, and cell-killing rates and EC₅₀ values were calculated. Results are shown in Table 5 below.

The results showed that IBR854 had significant killing effects on all eight tumor cell lines, with EC₅₀ (effector-to-target ratio) values between 0.86-5.06, and the maximum inhibition rate of above 75%.

**Table 5.**

| **Tumor cell line** | **Tumor cell passage** | **Date of IBR854 Lot** | **Mean EC₅₀ (effector-to-target ratio)** | **Maximum inhibition rate (%)** |
|---|---|---|---|---|
| H1975 | F13 | July 9, 2021 | 5.06 | 77.70 |
| NCI- H292 | F11 | September 11, 2021 | 0.86 | 100.44 |
| NCI-H226 | F15 | September 2, 2021 | 1.85 | 79.57 |
| HCC827 | F11 | August 4, 2021 | 1.47 | 103.98 |
| BxPC3 | F11 | July 13, 2021 | 4.33 | 88.19 |
| MDA-MB-231 | F9 | September 2, 2021 | 1.38 | 84.48 |
| MDA-MB-468 | F9 | September 13, 2022 | 2.91 | 79.77 |
| Panc-1 | F18 | August 15, 2022 | 2.48 | 76.71 |

### Example 4 - Evaluation of factor release function of conjugate IBR854

In this example, a functional study of cytokine secretion induced by conjugate IBR854 was performed. The investigated cytokines included IL-2 (interleukin-2), IL-6 (interleukin-6), IL-15 (interleukin-15), IL-1β (interleukin-1β), TNFα (tumor necrosis factor α), IFNγ (interferon γ), IL-8 (interleukin-8), CCL2 (chemokine 2), CCL3 (chemokine 3), CCL5 (chemokine 5), and the like. The measurement of cytokines was performed by using a commercial ELISA reagent kit and testing supernatants of various cell cultures according to the manufacturer's instructions.
(1) Conjugate IBR854 was co-incubated with three tumor effector cells (H1975, BxPC-3, and BxPC-3) at different effector-to-target ratios (1: 1, 3: 1, 10: 1, and 30:1). Secretion levels of IL-2, IL-6, IL-15, and IL-1β in cell culture supernatants were determined by ELISA. Results are shown in Table 6 to Table 9.
The results show that the secretion levels of IL-2, IL-6, IL-15, and IL-1β were all lower than the average levels in healthy individuals. IL-2 factor levels were all less than 2.0 pg/ml and in a dose-dependent manner, and were lower than the IL-2 level of 9.40±2.31 ng/ml in healthy human serum. IL-6 factor levels were all less than 0.3 pg/ml, and lower than the IL-6 level of 60.32±3.24 pg/ml in healthy human serum. IL-15 factor levels were all less than 0.5 pg/ml, and lower than the IL-15 level of 13.38±4.41 pg/ml in healthy human serum. The levels of IL-1β secretion were all less than 0.5 pg/ml.

**Table 6.**

| Effector-to-target ratio (IBR854: H1975) | Factor level (pg/ml) | | |
|---|---|---|---|
| | IL-2 | IL-6 | IL-15 |
| 30:1 | 1.27 | 0.09 | 0.34 |
| 10:1 | 0.481 | 0.076 | 0.2 |
| 3:1 | 0.417 | 0.196 | 0.003 |
| 1:1 | 0.062 | 0.029 | 0.008 |

**Table 7.**

| Effector-to-target ratio (IBR854: BXPC-3) | Factor level (pg/ml) | | |
|---|---|---|---|
| | IL-2 | IL-6 | IL-15 |
| 30:1 | 1.59 | 0.224 | 0.233 |
| 10:1 | 1.05 | 0 | 0.111 |
| 3:1 | 0.486 | 0.101 | 0.054 |
| 1:1 | 0.182 | 0.122 | 0.055 |

**Table 8.**

| Effector-to-target ratio (IBR854: Panc-1) | Factor level (pg/ml) | | |
|---|---|---|---|
| | IL-2 | IL-6 | IL-15 |
| 30:1 | 1.99 | 0 | 0.096 |
| 10:1 | 1.2 | 0.005 | 0.131 |
| 3:1 | 0.362 | 0 | 0 |
| 1:1 | 0.434 | 0.012 | 0.036 |

**Table 9.**

| Effector-to-target ratio (IBR854: H292) | IL-1β factor level (pg/ml) | | | |
|---|---|---|---|---|
| | June 9, 2022 | June 10, 2022 | June 15, 2022 | June 16, 2022 |
| 10:1 | 0.300 | 0.302 | 0.176 | 0.331 |
| 3:1 | 0.193 | 0.235 | 0.074 | 0.159 |
| 1:1 | 0.128 | 0.162 | 0.055 | 0 |

(2) Conjugate IBR854 was co-incubated with NCI-H292 and MDA-MB-231 tumor cells at effector-to-target ratios of 10:1, 3:1, and 1:1 (while a control group was set up to contain the conjugate IBR854 at the same effector-to-target ratios but without tumor cells). Secretion levels of TNFα and IFNγ in the culture supernatant were measured by ELISA. As shown by the results in Table 10 and Table 11, after the co-incubation of IBR854 with NCI-H292 and MDA-MB-231 tumor cells, the secretion levels of TNFα and IFNγ were positively correlated with the doses. The TNFα levels were lower than the average level in healthy individuals (22.75±6.28 µg/mL). After the co-incubation with tumor cells, the secretion levels of TNFα and IFNγ were significantly upregulated, indicating a certain correlation with cytotoxic effects on the tumors.

**Table 10.**

| Effector-to-target ratio (IBR854: NCI-H292) | Factor level (pg/ml) | |
|---|---|---|
| | TNFα | IFNγ |
| IBR854-10 | 7.59 | 442.78 |
| IBR854-3 | 1.83 | 126.31 |
| IBR854-1 | 0 | 43.65 |
| IBR854:NCI-H292-10:1 | 31.15 | 1009.40 |
| IBR854:NCI-H292-3:1 | 19.37 | 244.87 |
| IBR854:NCI-H292-1:1 | 9.45 | 69.84 |

**Table 11.**

| Effector-to-target ratio (IBR854: MDA-MB-231) | Factor level (pg/ml) | |
|---|---|---|
| | TNFα | IFNγ |
| IBR854-10 | 9.97 | 1011.78 |
| IBR854-3 | 0.48 | 302.58 |
| IBR854-1 | 0 | 103.93 |
| IBR854:MDA-MB-231-10:1 | 26.67 | 2341.82 |
| IBR854:MDA-MB-231-3:1 | 9.42 | 1447.22 |
| IBR854:MDA-MB-231-1:1 | 2.05 | 673.56 |

(3) Secretion levels of CCL2, CCL3, CCL5, and IL-8 (CXCL8) in the cell culture supernatants were determined by ELISA after the co-incubation of conjugate IBR854 with tumor cells H1975, BXPC-3, Panc-1 at different effector-to-target ratios (1:1, 3:1, 10:1, and 30:1). As shown by the results in Tables 12-14, the levels of CCL3, CCL5, and IL-8 (CXCL8) factors were positively correlated with exposure doses of IBR854. CCL3, CCL5, and IL-8 (CXCL8) were possible contributions to the tumor cell-killing effect of the IBR854 conjugate, which were achieved by mediating chemotaxis of related immune cells such as macrophages and leukocytes. The secretion levels of CCL2 were negatively correlated with the exposure doses of IBR854, possibly because the tumor cells were able to secrete CCL2 to achieve migration and invasion, while NK cells inhibited the chemotactic capability of the tumor cells, as evidenced by the decrease in CCL2 concentrations with increasing effector-to-target ratios of NK cells.

**Table 12.**

| Effector-to-target ratio (IBR854: H1975) | Factor level (pg/ml) | | |
|---|---|---|---|
| | CCL2 | CCL3 | CCL5 |
| 30:1 | 4.68 | 11785 | 6354 |
| 10:1 | 6.57 | 8466 | 4710 |
| 3:1 | 9.55 | 2937 | 1652 |
| 1:1 | 13.6 | 1113 | 639 |

**Table 13.**

| Effector-to-target ratio (IBR854: BXPC-3) | Factor level (pg/ml) | | |
|---|---|---|---|
| | IL-8 | CCL3 | CCL5 |
| 30:1 | 127 | 12916 | 7578 |
| 10:1 | 82.5 | 7085 | 4574 |
| 3:1 | 47.5 | 2905 | 1843 |
| 1:1 | 29.9 | 1018 | 655 |

**Table 14.**

| Effector-to-target ratio (IBR854: Panc-1) | Factor level (pg/ml) | | |
|---|---|---|---|
| | CCL2 | CCL3 | CCL5 |
| 30:1 | 75.9 | 2624 | 3964 |
| 10:1 | 492 | 873 | 1553 |
| 3:1 | 1928 | 236 | 664 |
| 1:1 | 2749 | 90.9 | 244 |

(4) Conjugate IBR854 was co-incubated with NCI-H292 and MDA-MB-231 tumor cells at different effector-to-target ratios of 10:1, 3:1, and 1:1 (while a control group was set up to contain the conjugate IBR854 at the same effector-to-target ratios but without tumor cells). Cell culture supernatants were collected and measured for secretion levels of granzyme B and perforin by ELISA. As shown by the results in Table 15 and Table 16, IBR854 alone secreted a certain amount of granzyme B and perforin in a dose-dependent manner. When IBR854 was co-incubated with NCI-H292 and MDA-MB-231 tumor cells, granzyme B expression levels were upregulated after tumor stimulation, while the secretion levels of perforin did not change significantly.

**Table 15.**

| Effector-to-target ratio (IBR854:NCI-H292) | Factor level (pg/ml) | |
|---|---|---|
| | Granzyme B | Perforin |
| IBR854-10 | 559.02 | 2681.84 |
| IBR854-3 | 93.72 | 792.66 |
| IBR854-1 | 53.45 | 365.77 |
| IBR854: NCI-H292-10:1 | 1035.16 | 2968.55 |
| IBR854: NCI-H292-3:1 | 244.35 | 853.34 |
| IBR854: NCI-H292-1:1 | 95.98 | 443.65 |

**Table 16.**

| Effector-to-target ratio (IBR854: MDA-MB-231) | Factor level (pg/ml) | |
|---|---|---|
| | Granzyme B | Perforin |
| IBR854-10 | 225.99 | 5390.77 |
| IBR854-3 | 55.53 | 2259.13 |
| IBR854-1 | 20.90 | 426.17 |
| IBR854: MDA-MB-231-10:1 | 428.46 | 6271.19 |
| IBR854: MDA-MB-231-3:1 | 56.40 | 2269.68 |
| IBR854: MDA-MB-231-1:1 | 18.98 | 608.24 |

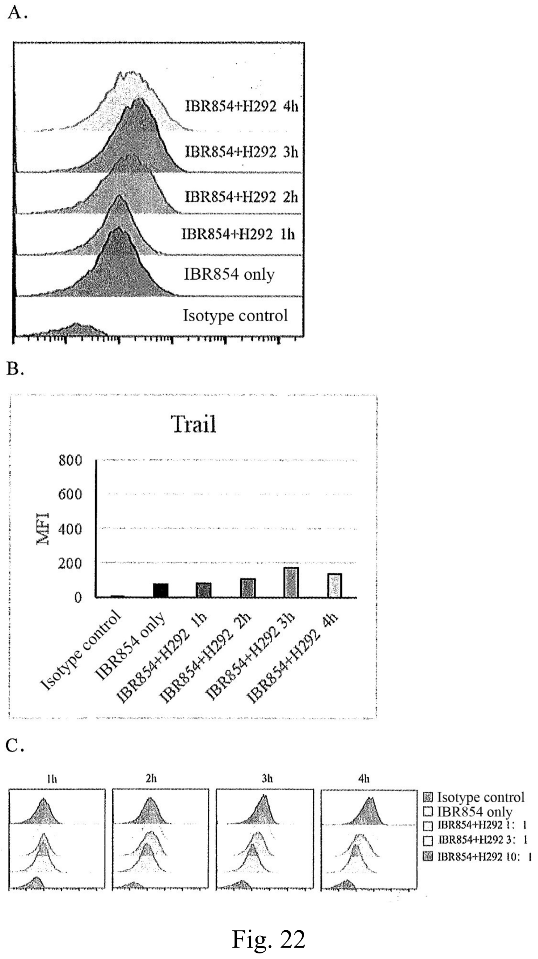

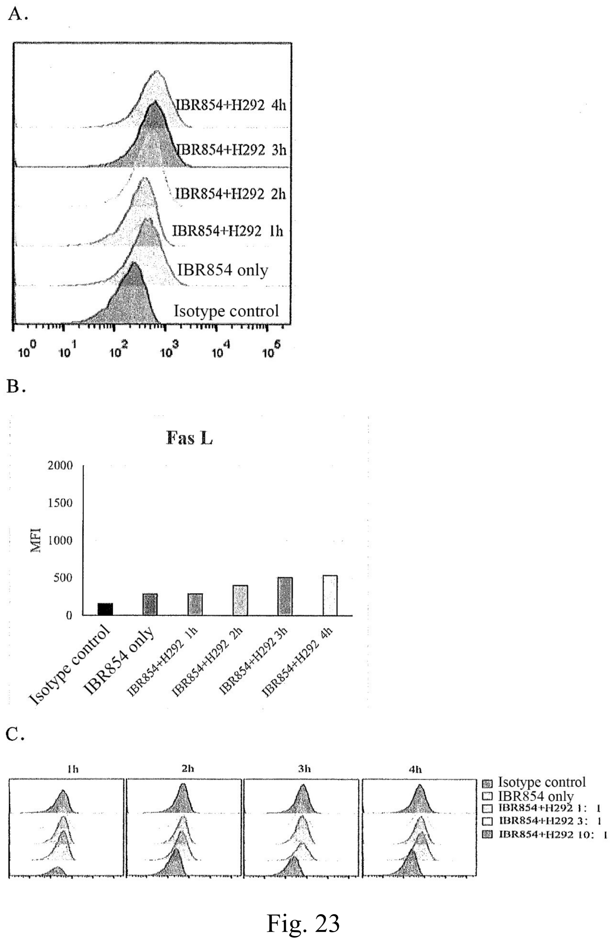

(5) Secretion levels of FasL/TRAIL factor after the co-incubation of IBR854 with H292 tumor cells were measured by flow cytometry. IBR854 and H292 tumor cells were co-incubated for 1 h, 2 h, 3 h, and 4 h, respectively, followed by staining with antibodies specific for TRAIL and FasL for flow cytometry. After the staining, expression levels of TRAIL and FasL on the surface of IBR854 cells were analyzed by flow cytometry. When H292 tumor cells were killed by IBR854, the expression of FasL and TRAIL on the surface of NK cells changed over time, increasing first and then decreasing, without a significant correlation with doses. Results are shown in FIG. 22 and FIG. 23.

### Example 5 - Pharmacodynamics study of conjugate IBR854 in NCI-H292 tumor-bearing mouse model

Seventy M-NSG mice were inoculated in each axilla with 5.0×10⁶ NCI-H292 cells. When the average tumor volume reached approximately 50 mm³, the animals were randomized into groups based on tumor volume. The experiment included 7 groups, with 10 mice per group:
Group 1 - Vehicle control (buffer for the formulation),
Group 2 - Antibody control (anti-5T4 antibody in Example 1, 0.75 mg/kg),
Group 3 - IL-15,
Group 4 - IL-15+IBR854 low dose (2.5×10⁸ cells/kg),
Group 5 - IL-15+IBR854 medium dose (5.0×10⁸ cells/kg),
Group 6 - IL-15+IBR854 high dose (1.0×10⁹ cells/kg),
Group 7 - IBR54 medium dose (5.0×10⁸ cells/kg).

In the *in vivo* pharmacodynamic assay, exogenous IL-15 was administered to prolong the persistence of IBR854 in the animals. IL-15 (90 µg/kg) was administered intraperitoneally once per week for a total of 3 doses. The remaining groups were administered via tail vein injection twice per week, with a one-day interval between doses, for a total of 6 doses. Body weight and tumor volume were measured twice per week. On Day 28, the M-NSG mice were sacrificed and tumor tissues were excised and weighed.

The design for the pharmacodynamic experiment in the mouse model with transplanted tumors of NCI-H292 human lung cancer is shown in Table 17.

**Table 17.**

| Group number | Group | Dose | Dosing volume (mL/kg) | Number of animals | Dosing route | Dosing regimen |
|---|---|---|---|---|---|---|
| 1 | Vehicle control | - | 20 | 10 | iv | BIW×3 W |
| 2 | Antibody control | 0.75 mg/kg | 20 | 10 | iv | BIW×3 W |
| 3 | IL-15 | 90 µg/kg | 0.4 mL/mouse | 10 | ip | QW×3 W |
| 4 | IL-15+IBR854 low dose | 2.5×10⁸ cells/kg | 20 | 10 | ip+iv | BIW×3 W |
| 5 | IL-15+IBR854 medium dose | 5.0×10⁸ cells/kg | 20 | 10 | ip+iv | BIW×3 W |
| 6 | IL-15+IBR854 high dose | 1.0×10⁹ cells/kg | 20 | 10 | ip+iv | BIW×3 W |
| 7 | IBR854 medium dose | 5.0×10⁸ cells/kg | 20 | 10 | iv | BIW×3 W |

| | | | | | | |
|---|---|---|---|---|---|---|
| -: Not applicable; BIW: twice a week; QW: once weekly; W: week; iv: intravenous injection; ip: intraperitoneal injection. | | | | | | |

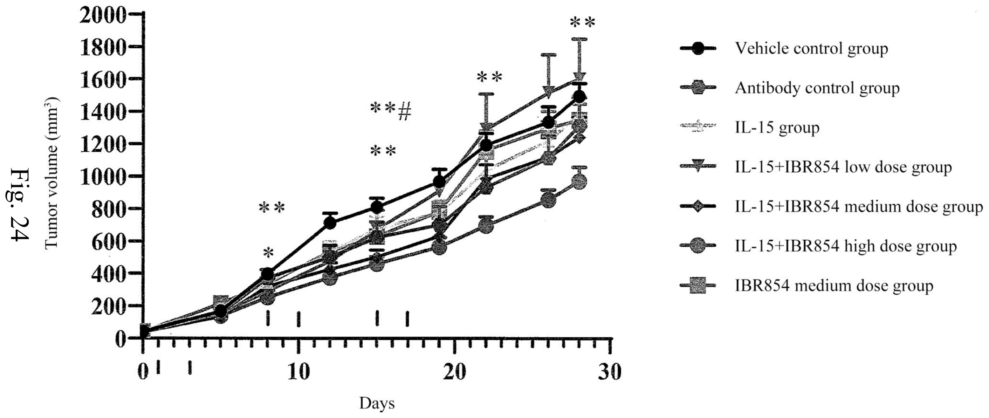

The results show the following.
1) At the end of the experiment, no death of mouse was observed in any group. Compared with the vehicle control group, no significant decrease in body weight was observed in mice from any IBR854 dose group, indicating good safety and tolerability of IBR854.
2) starting from Day 8, the tumor volume in each IL-15+IBR854 dose group showed varying degrees of reduction compared with the vehicle control group. By Day 28, the tumor volume, relative tumor volume (RTV), and relative tumor proliferation rate (T/C) in the IL-15+IBR854 high dose group were all significantly reduced compared with the vehicle control group, as shown in FIG. 24 and Table 18.

**Table 18.**

| Group | Tumor volume (mm³) (*x̅* ± *SE*) | | RTV (%) | T/C (%) | Tumor weight (g) (*x̅* ± *SE*) | IR (%) | |
|---|---|---|---|---|---|---|---|
| | Day 1 | Day 28 | | | | | |
| Vehicle control group | 42±4 | 1501±79 | 37.69±3.42 | - | 0.8951±0.1302 | - | |
| Antibody control group | 42±3 | 1318±174 | 33.04±5.36 | 87.66 | 0.8207±0.1435 | 8.31 | |
| IL-15 group | 42±3 | 1389±115 | 34.64±3.41 | 91.92 | 0.8311±0.1224 | 7.15 | |
| IL-15+IBR854 low dose group | 42±3 | 1608±246 | 40.05±6.85 | 106.26 | 0.9260±0.2169 | -3.45 | |
| IL-15+IBR854 medium dose group | 41±3 | 1246±126 | 29.93±1.52 | 79.41 | 0.7450±0.1296 | 16.77 | |
| IL-15+IBR854 high dose group | 42±3 | 975±86***## | 24.80±3.05* | 65.80 | 0.6807±0.0629 | 23.96 | |
| IBR854 group | medium dose | 41±3 | 1354±100 | 35.47±4.61 | 94.12 | 0.8611±0.0817 | 3.80 |

| | | | | | | | |
|---|---|---|---|---|---|---|---|
| -: Not applicable. *: p <0.05, **: p <0.01, ***: p <0.001, compared with the vehicle group; ##: p <0.01, compared with the IL-15 group; Tumor volume V=1/2×a×b², where a and b represent the long and short diameters of the tumor, respectively; Relative tumor volume (RTV) =Vt/V1, where V1 is the tumor volume measured at the time of dosing in groups (i.e., D1), and Vt is the tumor volume at each measurement; T/C (%) =TRTV/CRTV×100%, where TRTV is the RTV of the treated group, and CRTV is the RTV of the vehicle control group; IR (%) = (1-TWt/TWc) ×100%, where TWt is the tumor weight of the treated group, and TWc is the tumor weight of the vehicle control group. | | | | | | | |

Thus, under the experimental conditions of this example, animals treated with IB854 at doses ranging from 2.5×10⁸ to 10.0×10⁸ cells/kg were well tolerated. The administration of the high dose of IL-15+IBR854 via tail vein (twice weekly for a total of 6 doses) significantly inhibited tumor growth in the mice with transplanted tumor of human lung cancer cell NCI-H292.

### Example 6 - Pharmacodynamics study of conjugate IBR854 in MDA-MB-468 tumor-bearing mouse model

Fifty NCG mice were each inoculated with 4×10⁶ MDA-MB-468 cells. When the average tumor volume reached approximately 30-80 mm³, the animals were randomized into groups based on tumor volume. The experiment included 5 groups, with 10 mice per group:
Group 1 - Vehicle control (buffer for the formulation),
Group 2 - IL-15,
Group 3 - IL-15+IBR854 low dose (5×10⁸ cells/kg),
Group 4 - IL-15+IBR854 medium dose (1.0×10⁹ cells/kg),
Group 5 - IL-15+IBR854 high dose (1.5×10⁹ cells/kg).

In the *in vivo* pharmacodynamic assay, exogenous IL-15 was administered to prolong the persistence of IBR854 in animals. IL-15 (0.18 mg/kg) was administered intraperitoneally once per week for a total of 3 doses. The remaining groups were administered IBR854 via tail vein injection twice weekly for a total of 6 doses. Body weight and tumor volume were measured twice per week. On Day 20, the NCG mice were sacrificed and tumor tissues were excised and weighed.

The design of the pharmacodynamic experiment in the mouse model with transplanted tumors of human breast cancer cell MDA-MB-468 is shown in Table 19.

**Table 19.**

| Group number | Group | Dosage (cells/kg) | IL-15 dosage (mg/kg) | Number of animals | Dosing route | Dosing regimen |
|---|---|---|---|---|---|---|
| 1 | Vehicle control group | - | - | 10 | iv | BIW×3W |
| 2 | IL-15 group | - | 0.18 | 10 | ip | BIW×3W |
| 3 | IL-15+IBR854 low dose group | 5×10⁸ | 0.18 | 10 | ip+ iv | BIW×3W |
| 4 | IL-15+IBR854 medium dose group | 10×10⁸ | 0.18 | 10 | ip+ iv | BIW×3W |
| 5 | IL-15+IBR854 high dose group | 15×10⁸ | 0.18 | 10 | ip+ iv | BIW×3W |

| | | | | | | |
|---|---|---|---|---|---|---|
| -: Not applicable; Day 0 refers to the day of grouping for dosing; BIW: twice a week; W: week; iv: intravenous injection; ip: intraperitoneal injection. | | | | | | |

The results show the following.
1) Body weight and clinical observations. In this example, neither significant loss in body weight nor abnormalities in behaviors were observed in any one of mice, indicating that the mice exhibited good tolerance to the IBR854 injection under the experimental conditions.
2) Tumor volume. Mean tumor volume in Group 1 (vehicle control group) was 161.44±6.46 mm³ on day 20 of administration. Compared with the vehicle control group, the mean tumor volumes of Group 2 (IL-15 group), Group 3 (IL-15+IBR854 low dose group), Group 4 (IL-15+ IBR854 medium dose group), and Group 5 (IL-15+ IBR854 high dose group) were 160.18±11.6 mm³, 134.94±5.11 mm³ *(p* <0.05), 124.02±6.91 mm³ *(p* <0.001), and 114.19±3.50 mm³ *(p* <0.001), respectively. The tumor volumes in the IL-15+IBR854 low, medium, and high dose groups gradually decreased, showing a significant dose-dependent tumor inhibitory effect.
3) Tumor growth inhibition rate (TGI). Compared with the vehicle control group, the tumor growth inhibition rates (TGIs) of IL-15 group, IL-15+IBR854 low dose group, IL-15+IBR854 medium dose group, and IL-15+IBR854 low dose group were 0.74%, 31.49%, 44.11%, and 56.66%, respectively;
4) Tumor weights. Tumor weights were reduced by -6.00%, 16.85%, 22.97%, and 32.97%, respectively. Compared with the vehicle control group, volumes and weights of the tumors in each IBR854 treatment group were significantly reduced (*p* <0.05), demonstrating a clear and dose-dependent tumor-suppressive effect.

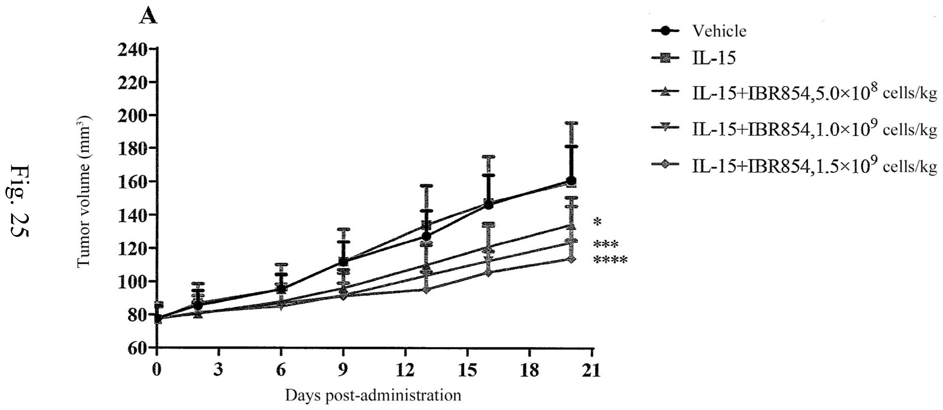

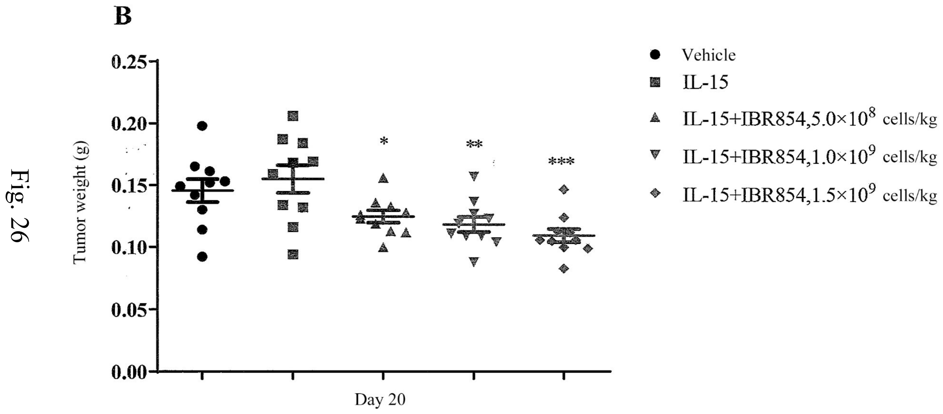

Specific results are shown in FIG. 25, FIG. 26 and Table 20.

**Table 20.**

| **Group number** | **Group** | **Tumor volume (mm3)** | | **TGI%** | **T/C%** | **Tumor weight (g)** |
|---|---|---|---|---|---|---|
| | | **Day 0** | **Day 20** | | | |
| 1 | Vehicle control | 77.80±2.39 | 161.44±6.46 | - | - | 0.146±0.009 |
| 2 | IL-15 | 77.16±3.01 | 160.18±11.36 | 0.74 | 99.26 | 0.155±0.011 |
| 3 | IL-15+IBR854 low dose group | 77.64±2.41 | 134.94±5.11* | 31.49 | 68.51 | 0.125±0.005* |
| 4 | IL-15+IBR854 total dose group | 77.27±3.09 | 124.02±6.91*** | 44.11 | 55.89 | 0.118±0.006** |
| 5 | IL-15+IBR854 high dose group | 77.94±2.11 | 114.19±3.50**** | 56.66 | 43.34 | 0.110±0.005*** |

| | | | | | | |
|---|---|---|---|---|---|---|
| ** p* <0.05, *** p* <0.01, **** p* <0.001, and **** *p* <0.0001, compared with the vehicle control group. Tumor volume V=0.5×a×b², where a and b represent the length and width of the tumor, respectively. Tumor growth inhibition rate TGI (%) = (1-T/C) ×100%. T/C% is the relative tumor proliferation rate, calculated as T/C% = (Tᵢ-T₀)/(Vᵢ-V₀) ×100%, that is, Ti is the average tumor volume at a time point after the start of administration of the treatment group, T₀ is the average tumor volume at the first administration of the treatment group, V₀ is the average tumor volume at the first administration of the vehicle control group, and Vi is the average tumor volume after the start of administration of the vehicle control group. T/C=T_{RTV}/C_{RTV}×100%, where T_{RTV} is the RTV of the treatment group and C_{RTV} is the RTV of the vehicle control group. | | | | | | |

Thus, under the experimental conditions of this example, animals treated with IB854 at doses ranging from 5.0×10⁸ to 1.5×10⁹ cells/kg were well tolerated. The administration of IL-15+IBR854 via tail vein (twice weekly for a total of 6 doses) significantly inhibited tumor growth in the mice with transplanted tumors of human breast cancer cell MDA-MB-468 in a dose-dependent manner.

### Example 7 - Evaluation of in vitro killing activities of anti-5T4 antibody-natural killer cell conjugates with different L linkers

An example of the linker conjugated to the antibody moiety in the anti-5T4 antibody-natural killer cell conjugate of the present application is dibenzoazacyclooctyne -glutaryl-amino polyethylene glycol -pentafluorophenyl acetylpiperidinate having the following structure: 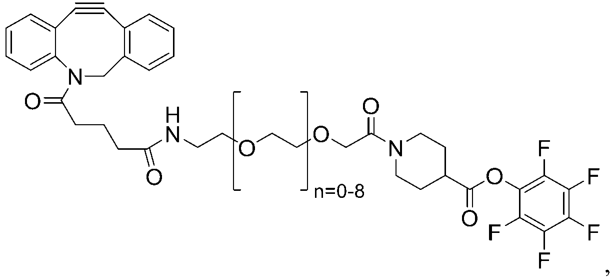 wherein n is an integer of 0-8.

In this example, *in vitro* killing activities of conjugates with two types of antibody linkers, L2 (n = 3) and L3 (n = 1) were tested.

L2 linker is the linker in IBR854 prepared in Example 1 above. Conjugate with L3 linker was prepared in a manner similar to that used for preparing IBR854 in Example 1, except that a different L2-2 substrate was used for synthesizing L3 linker. The corresponding substrate was also commercially available.

The assay for the *in vitro* killing activity was similar to that of Example 3. The test cell line was H292. The NK cells (obtained in Step (2) of Example 1) and the UNK cells (obtained in Step (6) of Example 1) were also selected as controls.

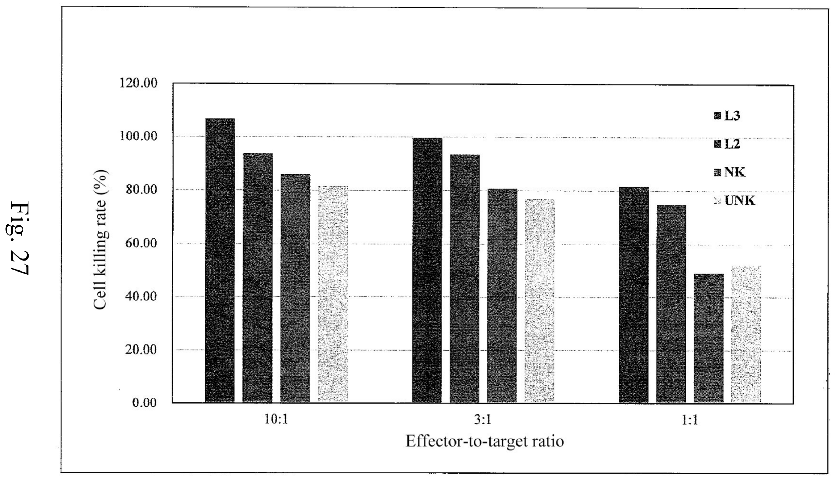

As shown by the results in FIG. 27, the conjugates with L2 and L3 linkers exhibited superior cytotoxicity against H292 cells compared with NK and UNK cells, and the conjugates with L2 (i.e., IBR854) and L3 linkers showed comparable killing activities.

### Example 8 - Evaluation of in vitro inhibitory activity of conjugate IBR854 on colorectal cancer and gastric cancer cells

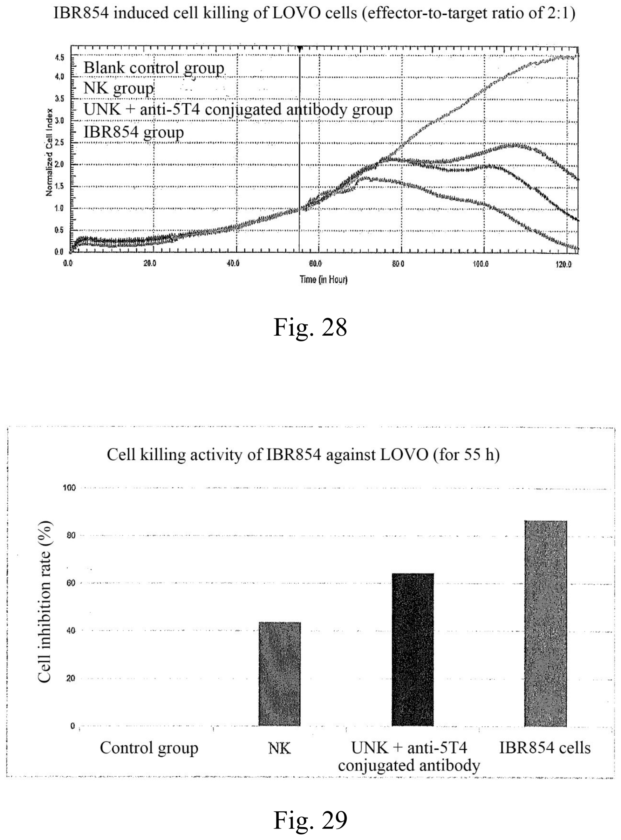

LOVO cells are colorectal cancer cells. LOVO cells were cultured in 96-well plates at 10000 cells/well and allowed to adhere. A study on cell inhibitory activity was performed with IBR854 (effector-to-target ratio of 2:1). The xCELLigence system was used to monitor tumor cell proliferation and changes in the inhibitory effect of effector cells (IBR854, and the NK cells or the mixture of UNK cells plus anti-5T4 antibody as controls) over time (FIG. 28). After 55 hours of treatment, the inhibition rate of NK cells was 43.6%, that of UNK+ anti-5T4 conjugated antibody (10 µg/ml) was 64.3%, and that of IBR854 was 86.8%. The control group did not contain any effector cells (FIG. 29).

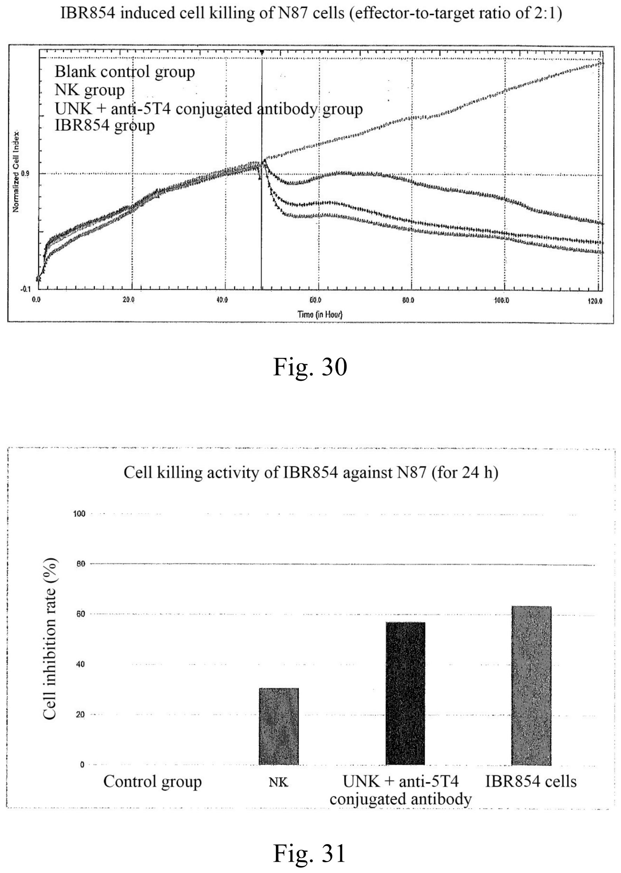

N87 cells are gastric adenocarcinoma cells. N87 cells were cultured in 8-well plates at 80000 cells/well and allowed to adhere. A study on cell inhibitory activity was performed with IBR854 (effector-to-target ratio of 2:1, for 24 hours). The xCELLigence system was used to monitor tumor cell proliferation and changes in the inhibitory effect of effector cells (IBR854, and the NK cells and the mixture of the UNK cell + the antibody as controls) over time (FIG. 30). After 24 hours of treatment, the inhibition rate of NK cells was 30.5%, that of UNK+ anti-5T4 conjugated antibody (10 µg/ml) was 56.9%, and that of IBR854 was 63.5% (FIG. 31).

The control group did not contain any effector cells.

The use of any and all embodiments or exemplary language (e.g., "such as") provided herein is intended only to better illustrate the present application and does not limit the scope of the present application unless otherwise required. The language in the specification should not be construed as indicating that any element not recited in a claim is necessary for the implementation of the inventions of the present application.

All publications and patent applications cited in the present specification are incorporated herein by reference as if each individual publication or patent application were specifically and individually identified by reference. Furthermore, any theory, mechanism, proof, or discovery described herein is intended to further facilitate the understanding of the present application and is not intended to limit the present application in any way to such theory, mechanism, proof, or discovery. Although the present application has been shown and described in detail in the drawings and the foregoing description, the present application should be considered illustrative and not limiting.

## Claims

1. An antibody-natural killer cell (NK cell) conjugate, wherein the antibody is an anti-5T4 antibody or antigen-binding fragment thereof, and the anti-5T4 antibody or antigen-binding fragment thereof is conjugated to the NK cell via a linker.

2. The antibody-natural killer cell (NK cell) conjugate of claim 1, wherein the anti-5T4 antibody or antigen-binding fragment thereof comprises:
HCDR1 as set forth in SEQ ID NO:5 or having at least 80%, 85%, 90%, 95% or 99% identity to the sequence set forth in SEQ ID NO:5,
HCDR2 as set forth in SEQ ID NO:6 or having at least 80%, 85%, 90%, 95% or 99% identity to the sequence set forth in SEQ ID NO:6,
HCDR3 as set forth in SEQ ID NO:7 or having at least 80%, 85%, 90%, 95% or 99% identity to the sequence set forth in SEQ ID NO:7,
LCDR1 as set forth in SEQ ID NO:8 or having at least 80%, 85%, 90%, 95% or 99% identity to the sequence set forth in SEQ ID NO:8,
LCDR2 as set forth in SEQ ID NO:9 or having at least 80%, 85%, 90%, 95% or 99% identity to the sequence set forth in SEQ ID NO:9, and
LCDR3 as set forth in SEQ ID NO:10 or having at least 80%, 85%, 90%, 95% or 99% identity to the sequence set forth in SEQ ID NO:10; and
wherein the amino acid sequences of the HCDRs and the LCDRs are defined according to Kabat.

3. The antibody-natural killer cell (NK cell) conjugate of claim 1 or 2, wherein the amino acid sequence of the heavy chain variable region of the anti-5T4 antibody or antigen-binding fragment thereof is set forth in SEQ ID NO:1 or has at least 80%, 85%, 90%, 95% or 99% identity to the sequence set forth in SEQ ID NO:1, and the amino acid sequence of the light chain variable region of the anti-5T4 antibody or antigen-binding fragment thereof is set forth in SEQ ID NO:3 or has at least 80%, 85%, 90%, 95% or 99% identity to the sequence set forth in SEQ ID NO:3.

4. The antibody-natural killer cell (NK cell) conjugate of any one of claims 1-3, wherein the anti-5T4 antibody comprises a heavy chain variable region set forth in SEQ ID NO:1, a heavy chain constant region set forth in SEQ ID NO:2, a light chain variable region set forth in SEQ ID NO:3, and a light chain constant region set forth in SEQ ID NO:4.

5. The antibody-natural killer cell (NK cell) conjugate of any one of claims 1-4, wherein:
the anti-5T4 antibody is an intact antibody, a single chain fragment variable (scFv) or a bispecific antibody; and/or
the antigen-binding fragment of the anti-5T4 antibody is a Fab, a Fab', a Fv or a F(ab')₂; and/or
the anti-5T4 antibody is a humanized antibody or a fully human antibody; and/or
the anti-5T4 antibody is a monoclonal antibody; and/or
the anti-5T4 antibody is of IgG1, IgG2 or IgG4 isotype; and/or
the anti-5T4 antibody comprises a light chain constant region of kappa subtype or lambda subtype.

6. The antibody-natural killer cell (NK cell) conjugate of any one of claims 1-5, wherein the NK cell is CD16⁺ and/or NKG2D⁺, preferably CD16⁺NKG2D⁺.

7. The antibody-natural killer cell (NK cell) conjugate of any one of claims 1-6, wherein the conjugate comprises at least 90% of CD16⁺NKG2D⁺NK cells, preferably, meanwhile the conjugate comprises at least 95% of CD56⁺NK cells.

8. The antibody-natural killer cell (NK cell) conjugate of any one of claims 1-7, wherein
the NK cell is obtained from *in vitro* culture and expansion of NK cells derived from peripheral blood mononuclear cells (PBMCs); or
the NK cell is obtained from *in vitro* culture and expansion of NK cells derived from umbilical cord blood; or
the NK cell is obtained from *in vitro* culture and expansion of an NK cell line; or
the NK cell is obtained from *in vitro* induction, culture, and expansion of induced pluripotent stem cells (iPSCs) or mesenchymal stem cells (ESCs).

9. The antibody-natural killer cell (NK cell) conjugate of any one of claims 1 to 8, wherein the anti-5T4 antibody or antigen-binding fragment thereof is conjugated to the NK cell via a click chemical reaction of linkers.

10. The antibody-natural killer cell (NK cell) conjugate of claim 9, wherein the anti-5T4 antibody or antigen-binding fragment thereof is conjugated to the NK cell via a first linker conjugated to the anti-5T4 antibody or antigen-binding fragment thereof, and a second linker conjugated to the NK cell, with the first and second linkers conjugated to each other to form the antibody-NK cell conjugate.

11. The antibody-natural killer cell (NK cell) conjugate of claim 10, wherein the first linker is an active ester capable of forming a conjugation to a lysine residue of the antibody by a reaction converting an ester bond into an amide bond, for example, the active ester is a pentafluorophenyl ester, such as pentafluorophenyl piperidinate.

12. The antibody-natural killer cell (NK cell) conjugate of claim 11, wherein the first linker further comprises a carbon-carbon triple bond structure capable of undergoing a cyclization reaction with an azide group to form a five-membered ring of triazole, for example, the carbon-carbon triple bond structure is an octyne group.

13. The antibody-natural killer cell (NK cell) conjugate of claim 12, wherein the first linker is a dibenzoazacyclooctyne-glutaryl-amino polyethylene glycol-pentafluorophenyl acetylpiperidinate having the following structure: 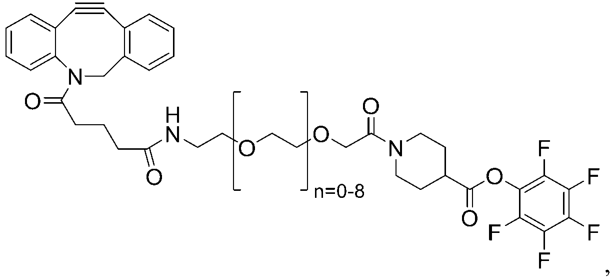 wherein n is an integer of 0-8.

14. The antibody-natural killer cell (NK cell) conjugate of claim 13, wherein the first linker is dibenzoazacyclooctyne-glutaryl-amino tetraethylene glycol-pentafluorophenyl acetylpiperidinate having the following structure:

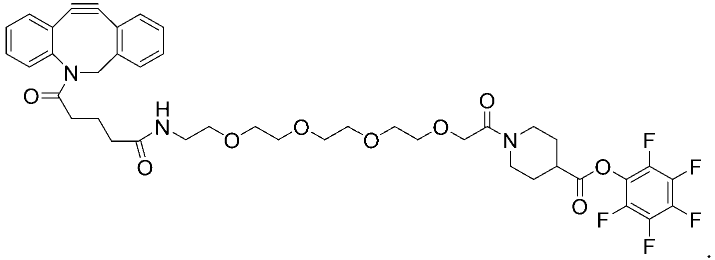

15. The antibody-natural killer cell (NK cell) conjugate of claim 10, wherein the second linker is an azidoacetylcyclohexosamine, such as an azidoacetylcyclogalactosamine or an azidoacetylglucosamine.

16. The antibody-natural killer cell (NK cell) conjugate of claim 15, wherein the second linker is 1,3,4,6-oxo-tetraacetyl-2-azidoacetamide-2-deoxy-a,b-D-galactose having the following structure:

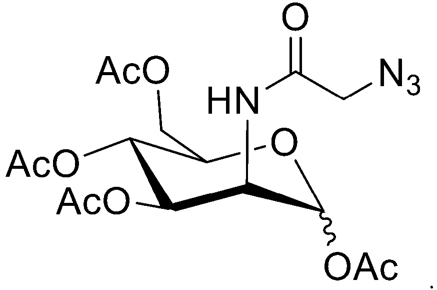

17. The antibody-natural killer cell (NK cell) conjugate of claim 16, wherein the second linker comprises at least 90% of the structure having a single α or β configuration.

18. A cell population comprising the antibody-natural killer cell (NK cell) conjugate of any one of claims 1-17.

19. The cell population of claim 18, wherein the count of CD3⁻CD56⁺CD16⁺ cells is at least 95%, preferably at least 98% with respect to the total cell count in the cell population, and/or the count of CD3⁻CD56⁺NKG2D⁺ cells is at least 95%, preferably at least 98% with respect to the total cell count in the cell population.

20. The cell population of claim 18 or 19, wherein:
the count of CD3⁺CD56⁺ cells is no more than 5% with respect to the total cell count in the cell population; and/or
the count of CD3⁻CD19⁺ cells is no more than 2% with respect to the total cell count in the cell population; and/or
the counts of CD3⁺CD4⁺ and CD3⁺CD8⁺ cells are no more than 2% with respect to the total cell count in the cell population.

21. The cell population of any one of claims 18-20, wherein, by cell count, the antibody-natural killer cell (NK cell) conjugate is at least 90%, preferably at least 95%, more preferably at least 98%, most preferably at least 99% with respect to the total cell count in the cell population.

22. A pharmaceutical composition comprising the antibody-natural killer cell (NK cell) conjugate of any one of claims 1-17, or the cell population of any one of claims 18-21, and a pharmaceutically acceptable carrier.

23. The pharmaceutical composition of claim 22, comprising sodium chloride and/or human serum albumin.

24. The pharmaceutical composition of claim 22 or 23, comprising trehalose, sucrose, dextran, DMSO, or any combination thereof.

25. A pharmaceutical composition of any one of claims 22-24, for use in the treatment of a tumor in an individual, particularly a tumor with a high expression of 5T4 (5T4+) in tumor cells.

26. The pharmaceutical composition of claim 25, wherein the tumor is a solid tumor.

27. The pharmaceutical composition of claim 25, wherein the tumor is a malignant tumor.

28. The pharmaceutical composition of claim 25, wherein the tumor is a cancer.

29. The pharmaceutical composition of claim 28, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, ovarian cancer, renal cancer, bladder cancer, cervical cancer, colon cancer, liver cancer, prostate cancer, peritoneal cancer, hepatocellular cancer, glioblastoma, urinary tract cancer, rectal cancer, endometrial or uterine cancer, salivary gland cancer, squamous cell cancer (e.g., squamous cell carcinoma), vulvar cancer, thyroid cancer, anal cancer, penile cancer, melanoma, B-cell lymphoma, brain cancer, head and neck cancer, and metastases of the above cancers.

30. The pharmaceutical composition of claim 28, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, and ovarian cancer.

31. The pharmaceutical composition of claim 28, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, and colorectal cancer.

32. Use of the antibody-natural killer cell (NK cell) conjugate of any one of claims 1-17 or the cell population of any one of claims 18-21 in the manufacture of a medicament for the treatment of a tumor in an individual, particularly a tumor with a high expression of 5T4 (5T4+) in tumor cells.

33. The use of claim 32, wherein the tumor is a solid tumor.

34. The use of claim 32, wherein the tumor is a malignant tumor.

35. The use of claim 32, wherein the tumor is a cancer.

36. The use of claim 35, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, ovarian cancer, renal cancer, bladder cancer, cervical cancer, colon cancer, liver cancer, prostate cancer, peritoneal cancer, hepatocellular cancer, glioblastoma, urinary tract cancer, rectal cancer, endometrial or uterine cancer, salivary gland cancer, squamous cell cancer (e.g., squamous cell carcinoma), vulvar cancer, thyroid cancer, anal cancer, penile cancer, melanoma, B-cell lymphoma, brain cancer, head and neck cancer, and metastases of the above cancers.

37. The use of claim 35, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, and ovarian cancer.

38. The use of claim 35, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, and colorectal cancer.

39. A method for treating a tumor in an individual, particularly a tumor with a high expression of 5T4 (5T4+) in tumor cells, the method comprising administering to the individual an effective amount of the antibody-natural killer cell (NK cell) conjugate of any one of claims 1-17, or the cell population of any one of claims 18-21, or the pharmaceutical composition of any one of claims 22-31.

40. The method of claim 39, wherein the tumor is a solid tumor.

41. The method of claim 39, wherein the tumor is a malignant tumor.

42. The method of claim 39, wherein the tumor is a cancer.

43. The method of claim 42, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, ovarian cancer, renal cancer, bladder cancer, cervical cancer, colon cancer, liver cancer, prostate cancer, peritoneal cancer, hepatocellular cancer, glioblastoma, urinary tract cancer, rectal cancer, endometrial or uterine cancer, salivary gland cancer, squamous cell cancer (e.g., squamous cell carcinoma), vulvar cancer, thyroid cancer, anal cancer, penile cancer, melanoma, B-cell lymphoma, brain cancer, head and neck cancer, and metastases of the above cancers.

44. The method of claim 42, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, colorectal cancer, and ovarian cancer.

45. The method of claim 42, wherein the cancer is selected from the group consisting of lung cancer (e.g., non-small cell lung cancer, small cell lung cancer, pulmonary adenocarcinoma, and pulmonary squamous cell carcinoma), breast cancer, pancreatic cancer, gastric cancer, and colorectal cancer.
